# EUROPEAN PATENT SPECIFICATION

(11) **EP 3 299 822 B1**
(45) Date of publication and mention of the grant of the patent: **05.12.2018**
(21) Application number: 17190370.1
(22) Date of filing: 10.11.2011
(51) Int. Cl.: G01N 33/92, G01N 33/68, G01N 33/94, G06F 19/00

(54) **METHOD AND USE OF METABOLIC COMPOUNDS FOR DIAGNOSING STROKE**
VERFAHREN UND VERWENDUNG VON METABOLISCHEN VERBINDUNGEN ZUR DIAGNOSE EINES ANFALLS
PROCÉDÉ ET UTILISATION DE COMPOSÉS MÉTABOLIQUES POUR DIAGNOSTIQUER UN ACCIDENT VASCULAIRE CÉRÉBRAL

(43) Date of publication of application: 28.03.2018
(62) Divisional of application: 11188592.7
(73) Proprietor: InfanDx AG, 50670 Köln (DE)
(72) Inventor: DEIGNER, Hans-Peter, 68623 Lampertheim (DE)
(74) Representative: Winter, Brandl, Fürniss, Hübner, Röss, Kaiser, Polte - Partnerschaft mbB

(56) References cited:
- WO-A2-2005/041893
- WO-A2-2007/127192
- WO-A2-2010/138899
- WO-A2-2011/106322
- PUIG N ET AL: "Serum Amino Acid Levels after Permanent Middle Cerebral Artery Occlusion in the Rat", CEREBROVASCULAR DISE, vol. 10, no. 6, 1 November 2000 (2000-11-01), pages 449-454, XP008175360, KARGER, BASEL, CH ISSN: 1015-9770, DOI: 10.1159/000016106 [retrieved on 2000-10-27]
- JOSÉ CASTILLO ET AL: "Biochemical Changes and Inflammatory Response as Markers for Brain Ischaemia: Molecular Markers of Diagnostic Utility and Prognosis in Human Clinical Practice", CEREBROVASCULAR DISEASES, vol. 17, no. 1, 1 December 2003 (2003-12-01), pages 7-18, XP055408920, CH ISSN: 1015-9770, DOI: 10.1159/000074791
- GLOBA O ET AL: "Stroke and epilepsy in children with inherited metabolic disorders", EUROPEAN JOURNAL OF NEUROLOGY, vol. 17, no. supplement 3, 1 September 2010 (2010-09-01), page 513, XP008149760, 14TH CONGRESS OF EUROPEAN-FEDERATION-OF-NEUROLOGICAL-SOCIET IES; GENEVA, SWITZERLAND; 201009, RAPID SCIENCE PUBLISHERS, GB ISSN: 1351-5101, DOI: 10.1111/J.1468-1331.2010.03233.X [retrieved on 2010-09-21]
- ZENENG WANG ET AL: "Targeted Metabolomic Evaluation of Arginine Methylation and Cardiovascular Risks: Potential Mechanisms Beyond Nitric Oxide Synthase Inhibition", ARTERIOSCLEROSIS, THROMBOSIS, AND VASCULAR BIOLOGY, vol. 29, no. 9, 1 September 2009 (2009-09-01), pages 1383-1391, XP008149006, LIPPINCOTT WILLIAMS & WILKINS, US ISSN: 1079-5642, DOI: 10.1161/ATVBAHA.109.185645 [retrieved on 2009-06-18]
- TUTTOLOMONDO A ET AL: "Plasma levels of inflammatory and thrombotic/fibrinolytic markers in acute ischemic strokes: Relationship with TOAST subtype, outcome and infarct site", JOURNAL OF NEUROIMMUNOLOGY, vol. 215, no. 1-2, 30 October 2009 (2009-10-30), pages 84-89, XP026682450, ELSEVIER SCIENCE PUBLISHERS BV ISSN: 0165-5728, DOI: 10.1016/J.JNEUROIM.2009.06.019 [retrieved on 2009-08-19]
- YURDAKOK MURAT ET AL: "Cerebrospinal fluid amino acid levels in newborn infants with intracranial hemorrhage", ACTA PAEDIATRICA JAPONICA, vol. 37, no. 6, 1 January 1995 (1995-01-01), pages 694-696, XP009147900, SOCIETAS PAEDIATRICA JAPONICA, JP ISSN: 0374-5600
- Maria Szpetnar ET AL: "The fluctuation of free amino acids in serum during acute ischemic stroke", Current Issues in Pharmacy and Medical Sciences, 1 December 2016 (2016-12-01), pages 151-154, XP055408928, DOI: 10.1515/cipms-2016-0031 Retrieved from the Internet: URL:https://www.degruyter.com/downloadpdf/ j/cipms.2016.29.issue-4/cipms-2016-0031/ci pms-2016-0031.pdf [retrieved on 2017-09-21]
- MATTHAN N R ET AL: "Specific Differences in the Predictive Value of Cholesterol Homeostatis Markers and 10-Year CVD Event Rate in Framingham Offspring Study", THE FASEB JOURNAL , vol. 27, no. 1, 847.19, 1 April 2013 (2013-04-01), XP002775914, Retrieved from the Internet: URL:http://www.fasebj.org/content/27/1_Sup plement/847.19.short [retrieved on 2017-11-23]

## Description

The present invention relates to a method for diagnosing stroke in accordance with claim 1, and a use of a plurality of naturally occurring metabolic compounds for diagnosing stroke from a patient's sample *in vitro,* according to claim 8.
The invention generally relates to biomarkers for stroke as tools in clinical diagnosis for early detection of stroke in a subject, and methods based on the same biomarkers.

### BACKGROUND of the Invention

Early detection of a disease based on symptoms as currently common in clinics is problematic since it is not reliable and the disease may have progressed before diagnosis is possible. Stroke is one such class of diseases and is assessed with diagnostic approaches.
Stroke, also known as cerebrovascular accident (CVA), is one of the leading causes of mortality and morbidity with an estimated 700,000 patients diagnosed with stroke each year in USA and in the EU, approximately 60.000 subjects. Stroke currently ranks third in the cause of death in the U.S.A. The term "stroke" encompasses two widely different clinical settings which it is of the utmost importance to distinguish. Ischemic stroke is thus usually caused by the blockage of blood vessels and is best treated by clot dissolving agents, such as t-PA, within three hours of symptom onset. In contrast, hemorrhagic stroke is caused by bleeding into the brain which forbids any treatment by anti-clotting agents, which could prove fatal.
As intended herein "stroke" relates to all cerebrovascular accidents. In particular, the term "stroke" encompasses acute and chronic stroke as well as ischemic and hemorrhagic stroke. Ischemic stroke is characterized by a partial or total occlusion of cerebral vessels which may lead to infarction and necrosis of cerebral tissues supplied by these vessels. In transient ischemic attack (TIA) the occlusion ceases spontaneously causing a dysfunction which lasts for no more than 24 hours. Hemorrhagic stroke is characterized by an intracerebral hemorrhage generally from cerebral vessel rupture.

Furthermore, the term "stroke" includes all stages of stroke(s).

An "onset of stroke" refers to an early stage of stroke, i.e., prior to a stage when the clinical manifestations are sufficient to support a clinical suspicion of stroke. The exact mechanism by which a patient acquires stroke is not a critical aspect of the invention. The methods of the present invention can detect changes in the biomarker score independent of the origin of the stroke in a subject. Regardless of how stroke in a subject arises, the methods of the present invention allow for determining the status of a patient having, or suspected of having stroke as classified by previously used criteria.

The diagnosis of stroke, and the segmentation between ischemic and hemorrhagic stroke, in patients which present with symptoms indicative of stroke, such as sudden numbness or blindness, confusion, severe headaches, slurred speech, and partial paralysis, currently essentially relies on **Computerized Tomography (CT) Scan,** which is generally the first diagnostic test done after a patient with a suspected stroke arrives in the emergency room. The test involves the use of low-dose x-rays to visualize the brain. CT, however, is not completely satisfying since it has an estimated sensitivity of less than 26% in diagnosing acute stroke (Chalela et al. (2007) Lancet 369:293-298), which is linked to a very poor performance in detecting ischemic stroke, with less than 33% sensitivity (Reynolds et al. (2003) Clin. Chem. 49:1733-1739). **Magnetic Resonance Imaging (MRI)** is an advanced diagnostic tool that provides a high level of anatomic detail for precisely locating the stroke and determining the extent of damage. Due to its high level of sensitivity, MRI is considered especially useful when the stroke involves small blood vessels. The technology involves use of a strong magnetic field, and is performed in a special room free of metallic equipment. Recently, there have been great advances in the early detection of stroke using diffusion and perfusion weighted imaging. Magnetic resonance imaging (MRI) has been shown to be superior to CT in diagnosing acute stroke (84% sensitivity, Chalela et al. (2007) Lancet 369:293-298), and particularly ischemic stroke. However MRI scanners are costly equipments and are not always available in the emergency room. **Magnetic Resonance Angiography (MRA)** is a new noninvasive technology for imaging the cerebral blood vessels, which yields valuable information regarding collateral (alternative) blood vessels in the brain. **Carotid Duplex Scanning** is a noninvasive study to diagnose blockage in the carotid arteries. This technology involves recording sound waves that reflect the velocity of blood flow. **Transcranial Doppler (TCD)** is a new, noninvasive ultrasound procedure that allows the assessment of blood flow through the cerebral vessels via a small probe placed against the skull. TCD is a portable test, which can be performed frequently at the patient's bedside to follow the progress of medical treatment for stroke. **Xenon CT Scanning** is an imaging method that uses the inert gas xenon to measure blood flow in various brain regions. **Radionuclide SPECT Scanning** provides data on relative blood flow using the radionuclide Technetium-99. **PET Scanning,** which measures brain cell metabolism, can determine if brain tissue is functioning even if blood flow to that area appears to be diminished. **Cerebral Angiography (angiogram)** is a diagnostic method that requires injection of a contrast dye through a major artery (usually the femoral artery in the thigh) for evaluation of blood flow to the brain. This procedure is completed in Stanford's Cath/Angio lab. The procedure time is approximately two to three hours; bed rest for six hours is required after the procedure. **Transesophageal Echocardiography** involves placing a flexible tube in the oesophagus to directly image the heart. These diagnostic approaches, however, all rely on expensive and sophisticated instrumentation and require long turnaround times. For diagnosis of acute stroke, however, they have limitations since, unlike events on the metabolite level, they do not indicate an acute or recent lesion. Therefore, issues detected by MRT or above mentioned techniques may indicate a preceding event completely unrelated to acute disorders. Even more important is that none of these technologies allow diagnosis or confirmation of stroke on site and far from a hospital or GP. So you can say that current diagnostic tests to evaluate whether a subject had stroke have major limitations with low sensitivity and specificity.
Accordingly there is still the need for alternative or complementary methods for diagnosing stroke. Due to these technical limitations academic research groups have been looking for potential alternative biomarkers of stroke. Various markers for stroke are known and analytical techniques for the determination of such markers have been described in the art. Known biomarker for stroke and stroke related brain include diagnostic protein marker comprising e.g. interleukin 17, Fas ligand, nerve growth factor, insulin growth factor binding protein 3, p55, tumor necrosis factor receptor (TNFR-1), growth-related oncogene alpha, interleukin-2 soluble receptor gamma and combinations thereof (WO 2010/0318).

As used herein the term "marker" or biomarker refers to typically naturally occurring metabolic compounds which are present in body liquid and/or are being released from the brain during a cerebral ischemic or hemorrhagic event. Known marker and agents described in the context of stroke, however, include single protein marker with unsatisfying sensitivities and specificities, also not used as marker combinations or combined marker panels. For instance, it has been reported in the literature that myelin basic protein (MBP) concentration, in cerebrospinal fluid (CSF) increases after sufficient damage to neuronal tissue, head trauma and AIDS dementia. See Strand, T., et al., Brain and plasma proteins in spinal fluid as markers for brain damage and severity of stroke, Stroke (1984) 15; 138-144. S100 protein is another marker which may be taken as a useful marker for assessing neurologic damage and for determining the extent of brain damage and for determining the extent of brain lesions. Thus, it has been suggested for use as an aid in the diagnosis and assessment of brain lesions and neurological damage due to stroke. See Missler, U., Weismann, M., Friedrich, C. and Kaps, M., S100 protein and neuron-specific enolase concentrations in blood as indicators of infarction volume and prognosis in acute ischemic stroke (Stroke (1997) 28; 1956-60). Neuron-specific enolase (NSE) also has been suggested as a useful marker of neurologic damage in the study of stroke with particular application in the assessment of treatment (see Teasdale, G. and Jennett, B., Assessment of coma and impaired consciousness, Lancet (1974) 2; 81-84) or compounds that bind translocator protein (18kDa) (TSPO) and methods for imaging TSPO expression in a subject (WO2010020000 / 2010-25-25). Heart-type fatty acid binding protein (H-FABP) has also been considered as a promising marker (Lescuyer et al. (2005) Mol. Diagn. 9:1 - 7). However, it seems that the discriminatory power offered by these markers individually is not sufficient to be of clinical value. It has thus been suggested to use panels combining several markers, such as S-100b, the B-type neurotrophic growth factor (BNGF), the von Willebrand factor (vWF), matrix metalloproteinase-9 (MMP-9) and monocyte chemotactic protein-1 (MCP-1), for diagnosing ischemic stroke (Reynolds et al. (2003) Clin. Chem. 49:1733-1739). This panel was shown to provide a sensitivity of 92% at 93% specificity for ischemic stroke sample within 6 hours from symptom onset. Within 3 hours from onset however, sensitivity is of only 87%, which might be due to a too low individual sensitivity/specificity of the markers. Accordingly, there is still the need for alternative markers offering a good individual sensitivity/specificity ratio to be used in single-marker tests or to improve multi-marker panels, either by increasing sensitivity/specificity or by enabling reducing the number of markers which levels have to be measured in panels.

Advantageously, the method of the invention provides for early stroke diagnosis. Early stroke diagnosis is of particular importance in the case of ischemic stroke, since it is usually estimated that treating the occluded vessels within 3 hours of stroke symptoms onset will prevent most irreversible cerebral damages. For stroke therapy, however, time to therapy is highly critical to avoid or limit permanent brain damage. For instance for streptokinase therapy, poor outcomes were confined to patients receiving therapy more than 3 hours after stroke onset. In contrast, among the 70 patients who were entered into the trial within 3 hours of stroke onset, there was a trend toward improved outcomes for those who received streptokinase and this outcome pattern was significantly better than for those receiving therapy after 3 hours JAMA. 1996;276(12):961-966*.* Accordingly, it is preferred that the above defined step (a) is implemented within 6 hours, more preferably within 3 hours, and most preferably within 2 hours, after the onset of at least one symptom indicative of stroke in the individual. The symptoms indicative of stroke are well known to one of skill in the art and notably encompass sudden numbness or blindness, confusion, severe headaches, slurred speech, and partial paralysis.

In summary there is no reliable biomarker available to diagnose or predict hypoxic-ischemic stroke at an early stage. Currently used diagnostic methods thus require time and appropriate equipment with high costs and frequently unsatisfying sensitivities. However, these used diagnostic means have major limitations such as reduced area under the curve (AUC) and/or delay of diagnosis or increased costs due to equipment required. Accordingly, these procedures do not allow a timely assessment of an acute and rapidly evolving disease nor a differentiation of brain areas affected. Overall the situation is far from satisfying and from providing a rapid, reliable and precise diagnosis of stroke as a prerequisite for timely and adequate treatment.

In classical patient screening and diagnosis, the medical practitioner uses a number of diagnostic tools for diagnosing a patient suffering from a certain disease. Among these tools, measurement of a series of single routine parameters, e.g. in a blood sample, is a common diagnostic laboratory approach. These single parameters comprise for example enzyme activities and enzyme concentration and/or enzyme detection
As far as such diseases are concerned which easily and unambiguously can be correlated with one single parameter or a few number of parameters achieved by clinical chemistry, these parameters have proved to be indispensable tools in modern laboratory medicine and diagnosis. However, in complex pathophysiological conditions, which share a lack of an unambiguously assignable single parameter or marker, differential diagnosis from blood or tissue samples is currently difficult to impossible.
WO 2010/128054 A1 relates to a method for in vitro diagnosing e.g. perinatal asphyxia and disorders related to hypoxia.
Stroke and asphyxia, however, are completely different medical entities. Whereas asphyxia or hypoxia denote a generalized and global deprivation of oxygen of an organism with changes affecting the whole body and many organs, ischemic stroke is a locally confined event causing a local cerebral lesion with a clear focus and subsequent changes of biological conditions in the brain area of the insult only, due to occlusion (ischemic stroke) or bleeding (hemorrhagic stroke). Therefore, no conclusions on biochemical alterations or metabolites involved in post-stroke events are possible based on data obtained from subjects with asphyxia. (see P Govaert, L Ramenghi, R Taal, L De Vries, G DeVeber: Acta Paediatrica Volume 98, Issue 10, pages 1556-1567, October 2009: "Diagnosis of perinatal stroke I: definitions, differential diagnosis and registration").
Solberg R, Enot, D, Deigner, H-P, Koal, T, Scholl-Bürgi, S, Saugstad, OD, and Keller, M (2010): "Metaboloic Analyses of Plasma Reveals New Insights into Asphyxia and Resuscitation in Pigs", PLoS ONE 5(3): e9606. doi:10.1371/journal.pone.0009606 disclose detection of a number of metabolites in plasma taken before and after hypoxia as well as after resuscitation, in asphyxiated mice, in order to evaluate pathophysiological mechanisms of hypoxemia in newborns. The metabolites of the study of Solberg et al. [2010] include amino acids, particularly branched chained amino acids, metabolites of the Krebs cycle, including α-ketoglutarate, succinate and fumarate, biogenic amines, bile acids, prostaglandins, sphingolipids, glycerophospholipids, oxysterols and acylcarnitines.
Mueller et al. [2007] investigated in a group of subject a number of 65 quantitatively detected metabolites (42 organic acids, 22 acylcarnitines, free carnitine and 15 ratios) in urine within the first 72 hours of life of subject. Reliable prediction for development of HIE (hypoxic-ischemic encephalopathy) caused by severe asphyxia was demonstrated with metabolite monitoring of the lactic acid/creatinine ratio in urine of asphyxiated newborns.
However, an unexpected result of the Mueller et al. [2007] study was the finding that the total amount of urinary acylcarnitines differed not significantly between the comparison group and the patient group with severe neurological defects.
However, while stroke goes along with conditions of asphyxia, it clearly is a separate medical entity defined as a rapidly developing loss of brain function(s) due to disturbance in the blood supply to the brain. This can be due to ischemia (lack of blood flow) caused by blockage (thrombosis, arterial embolism), or a hemorrhage (leakage of blood) [Sims NR, Muyderman H (September 2009). "Mitochondria, oxidative metabolism and cell death in stroke". Biochimica et Biophysica Acta 1802 (1): 80-91]. A stroke thus is caused by the interruption of the blood supply to the brain, usually because a blood vessel bursts or is blocked by a clot. This cuts off the supply of oxygen and nutrients, causing damage to the brain tissue. As a result, the affected area of the brain is unable to function, leading to inability to move one or more limbs on one side of the body, inability to understand or formulate speech, or an inability to see one side of the visual field.[Donnan GA, Fisher M, Macleod M, Davis SM (May 2008). "Stroke". Lancet 371 (9624): 1612-23]. Puig et al. (2000), Cerebrovasc Dis: 10-449-454 disclose a method for measuring amino acid levels in serum to diagnose acute stroke. So far, no metabolic markers have been introduced for indicating and diagnosing stroke in a subject. Solely a couple of intermediates, possibly involved in pathobiochemistry, have been discussed.
Thus, it is the problem underlying the present invention to provide a diagnostic approach for stroke in a subject.
Given the remarkable and rapid potential of stroke in subject to progress into an irreversible and life-threatening condition the current situation is highly problematic and unsatisfying and an early and reliable multi-parameter diagnosis based on small sample sizes of body liquids is imperative to appropriate therapy such as streptokinase administration outlined above.

The above mentioned problems are solved by a method in accordance with claim 1, and a use according to claim 8.

The current invention presents a solution to this problem ideally requiring only minute amounts of blood.

In particular, the present disclosure relates to a method for diagnosing stroke (cerebrovascular accident) in patients characterized by quantitatively detecting in vitro in at least one biological sample of at least one tissue of a patient a plurality of at least two metabolic compounds being specific for stroke, and having a molecular weight of less than 1500 Dalton comprising the steps of:
a) selecting said metabolic compounds from the group consisting of the compounds of the following table:

| **Metabolic Compound** | **Abbreviation** |
|---|---|
| Carnitine | C0 |
| Propionylcarnitine | C3 |
| Butyrylcarnitine | C4 |
| Valerylcarnitine | C5 |
| Tetradecanoylcarnitine | C14 |
| Octadecanoylcarnitine | C18 |
| Hydroxyvalerylcarnitine/Methylmalonylcarnitine | C5-OH/ C3-DC-M |
| 4β-Hydroxycholesterol | 4b-OH-C |
| Lanosterol | |
| Desmosterol | |
| Arginine | Arg |
| Asparaginic acid | Asp |
| Tyrosine | Tyr |
| Ornithine | Orn |
| Threonine | Thr |
| Phenylalanine | Phe |
| Serine | Ser |
| Glutamine | Glu |
| Arachidonic acid | AA |
| Serotonin | |
| Phospatidylcholine acyl-alkyl C42:1 | PC ae C42:1 |
| Phospatidylcholine acyl-alkyl C40:1 | PC ae C40:1 |
| Phospatidylcholine acyl-alkyl C36:3 | PC ae C36:3 |
| Phospatidylcholine acyl-alkyl C36:4 | PC ae C36:4 |
| Phospatidylcholine acyl-alkyl C36:5 | PC ae C36:5 |
| Phospatidylcholine acyl-alkyl C38:4 | PC ae C38:4 |
| Phospatidylcholine acyl-alkyl C38:5 | PC ae C38:5 |
| Phospatidylcholine diacyl C42:1 | PC aa C42:1 |
| Phospatidylcholine diacyl C34:4 | PC aa C34:4 |
| Lysophospatidylcholine acyl C24:0 | lysoPC a C24:0 |
| Lysophospatidylcholine acyl C16:1 | lysoPC a C16:1 |
| Lysophospatidylcholine acyl C14:0 | lysoPC a C14:0 |
| Lysophospatidylcholine acyl C20:3 | lysoPC a C20:3 |
| Lysophospatidylcholine acyl C17:0 | lysoPC a C17:0 |
| Lysophospatidylcholine acyl C18:1 | lysoPC a C18:1 |
| Lysophospatidylcholine acyl C18:0 | lysoPC a C18:0 |
| Lysophospatidylcholine acyl C16:0 | lysoPC a C16:0 |
| Lysophospatidylcholine acyl C16:1 | lysoPC a C16:1 |
| Glycocholic acid | GCA |
| Glycochenodeoxycholic acid | GCDCA |
| Chenodeoxycholic acid | CDCA |
| Glycolithocholic acid | GLCA |
| Glycolithocholic acid sulfate | GLCAS |
| Taurocholic acid | TCA |
| Taurolithocholic acid | TLCA |
| Taurine-conjugated Taurochenodeoxycholic acid | TCDCA |
| Taurolithocholic acid sulfate | TLCAS |
| Taurodeoxycholic acid | TDCA |
| Docosahexaenoic acid | DHA |

wherein in acylcarnitines, phosphatidylcholines, and lysophosphatidylcholines, the number following "C" represents the number of total carbon atoms in the fatty acid and/or alkyl residue(s), and the number after the colon represents the number of total double bonds in the fatty acid residue and/or alkyl residue(s);
b) measuring at least one of the parameters selected from the group consisting of: presence, concentration, level or amount of each specific compound of said plurality of compounds in said sample; obtaining a qualitative and/or quantitative molecular pattern from the entirety of the obtained values from said parameter measurements; and storing said obtained set of values in a database;
c) calibrating said values by comparing clinically confirmed stroke-positive and/or clinically confirmed stroke-negative reference parameters; and
d) comparing said measured values in the sample with the calibrated values, in order to assess whether the patient is stroke-positive or stroke-negative.

Furthermore, the present disclosure is directed to a use of a plurality of at least two metabolic compounds for diagnosing stroke from a biological sample in vitro, wherein said compounds have a molecular weight of less than 1500 Dalton, and wherein said compounds are selected from the group consisting of the following table:

| **Metabolic Compound** | **Abbreviation** |
|---|---|
| Carnitine | C0 |
| Propionylcarnitine | C3 |
| Butyrylcarnitine | C4 |
| Valerylcarnitine | C5 |
| Tetradecanoylcarnitine | C14 |
| Octadecanoylcarnitine | C18 |
| Hydroxyvalerylcarnitine/ Methylmalonylcarnitine | C5-OH/ C3-DC-M |
| 4β-Hydroxycholesterol | 4b-OH-C |
| Lanosterol | |
| Desmosterol | |
| Arginine | Arg |
| Asparaginic acid | Asp |
| Tyrosine | Tyr |
| Ornithine | Orn |
| Threonine | Thr |
| Phenylalanine | Phe |
| Serine | Ser |
| Glutamine | Glu |
| Arachidonic acid | AA |
| Serotonin | |
| Phospatidylcholine acyl-alkyl C42:1 | PC ae C42:1 |
| Phospatidylcholine acyl-alkyl C40:1 | PC ae C40:1 |
| Phospatidylcholine acyl-alkyl C36:3 | PC ae C36:3 |
| Phospatidylcholine acyl-alkyl C36:4 | PC ae C36:4 |
| Phospatidylcholine acyl-alkyl C36:5 | PC ae C36:5 |
| Phospatidylcholine acyl-alkyl C38:4 | PC ae C38:4 |
| Phospatidylcholine acyl-alkyl C38:5 | PC ae C38:5 |
| Phospatidylcholine diacyl C42:1 | PC aa C42:1 |
| Phospatidylcholine diacyl C34:4 | PC aa C34:4 |
| Lysophospatidylcholine acyl C24:0 | lysoPC a C24:0 |
| Lysophospatidylcholine acyl C16:1 | lysoPC a C16:1 |
| Lysophospatidylcholine acyl C14:0 | lysoPC a C14:0 |
| Lysophospatidylcholine acyl C20:3 | lysoPC a C20:3 |
| Lysophospatidylcholine acyl C17:0 | lysoPC a C17:0 |
| Lysophospatidylcholine acyl C18:1 | lysoPC a C18:1 |
| Lysophospatidylcholine acyl C18:0 | lysoPC a C18:0 |
| Lysophospatidylcholine acyl C16:0 | lysoPC a C16:0 |
| Lysophospatidylcholine acyl C16:1 | lysoPC a C16:1 |
| Glycocholic acid | GCA |
| Glycochenodeoxycholic acid | GCDCA |
| Chenodeoxycholic acid | CDCA |
| Glycolithocholic acid | GLCA |
| Glycolithocholic acid sulfate | GLCAS |
| Taurocholic acid | TCA |
| Taurolithocholic acid | TLCA |
| Taurine-conjugated Taurochenodeoxycholic acid | TCDCA |
| Taurolithocholic acid sulfate | TLCAS |
| Taurodeoxycholic acid | TDCA |
| Docosahexaenoic acid | DHA |

wherein in acylcarnitines, phosphatidylcholines, and lysophosphatidylcholines, the number following "C" represents the number of total carbon atoms in the fatty acid and/or alkyl Residue(s), and the number after the colon represents the number of total double bonds in the fatty acid residues and/or alkyl residue(s);

Another aspect of the present disclosure is to provide biomarkers for diagnosing stroke (cerebrovascular accident), characterized by comprising a plurality of at least two metabolic compounds being specific for stroke, which are selected from the group consisting of the compounds of the following table:

| **Metabolic Compound** | **Abbreviation** |
|---|---|
| Carnitine | C0 |
| Propionylcarnitine | C3 |
| Butyrylcarnitine | C4 |
| Valerylcarnitine | C5 |
| Tetradecanoylcarnitine | C14 |
| Octadecanoylcarnitine | C18 |
| Hydroxyvalerylcarnitine/ Methylmalonylcarnitine | C5-OH/ C3-DC-M |
| 4β-Hydroxycholesterol | 4b-OH-C |
| Lanosterol | |
| Desmosterol | |
| Arginine | Arg |
| Asparaginic acid | Asp |
| Tyrosine | Tyr |
| Ornithine | Orn |
| Threonine | Thr |
| Phenylalanine | Phe |
| Serine | Ser |
| Glutamine | Glu |
| Arachidonic acid | AA |
| Serotonin | |
| Phospatidylcholine acyl-alkyl C42:1 | PC ae C42:1 |
| Phospatidylcholine acyl-alkyl C40:1 | PC ae C40:1 |
| Phospatidylcholine acyl-alkyl C36:3 | PC ae C36:3 |
| Phospatidylcholine acyl-alkyl C36:4 | PC ae C36:4 |
| Phospatidylcholine acyl-alkyl C36:5 | PC ae C36:5 |
| Phospatidylcholine acyl-alkyl C38:4 | PC ae C38:4 |
| Phospatidylcholine acyl-alkyl C38:5 | PC ae C38:5 |
| Phospatidylcholine diacyl C42:1 | PC aa C42:1 |
| Phospatidylcholine diacyl C34:4 | PC aa C34:4 |
| Lysophospatidylcholine acyl C24:0 | lysoPC a C24:0 |
| Lysophospatidylcholine acyl C16:1 | lysoPC a C16:1 |
| Lysophospatidylcholine acyl C14:0 | lysoPC a C14:0 |
| Lysophospatidylcholine acyl C20:3 | lysoPC a C20:3 |
| Lysophospatidylcholine acyl C17:0 | lysoPC a C17:0 |
| Lysophospatidylcholine acyl C18:1 | lysoPC a C18:1 |
| Lysophospatidylcholine acyl C18:0 | lysoPC a C18:0 |
| Lysophospatidylcholine acyl C16:0 | lysoPC a C16:0 |
| Lysophospatidylcholine acyl C16:1 | lysoPC a C16:1 |
| Glycocholic acid | GCA |
| Glycochenodeoxycholic acid | GCDCA |
| Chenodeoxycholic acid | CDCA |
| Glycolithocholic acid | GLCA |
| Glycolithocholic acid sulfate | GLCAS |
| Taurocholic acid | TCA |
| Taurolithocholic acid | TLCA |
| Taurine-conjugated Taurochenodeoxycholic acid | TCDCA |
| Taurolithocholic acid sulfate | TLCAS |
| Taurodeoxycholic acid | TDCA |
| Docosahexaenoic acid | DHA |

wherein in acylcarnitines, phosphatidylcholines, and lysophosphatidylcholines, the number following "C" represents the number of total carbon atoms in the fatty acid and/or alkyl residue(s), and the number after the colon represents the number of total double bonds in the fatty acid residue and/or alkyl residue(s);
Generally, in accordance with the present invention, the following metabolites with their below given chemical characterizations were detected: Amino acids, acylcarnitines [Cx:y with x being the chain length and y being the number of double bonds], hydroxyacylcarnitines [C(OH)x:y], dicarboxyacylcarnitines [Cx:y-DC], free Carnitin [C0], biogenic amines, hexoses, and lipids.

The lipids used for the present invention are subdivided into different classes of lipids: Glycerophospholipids: The basic structure of glycerophospholipids comprises a glycerol moiety, two of its hydroxy groups being bound to two fatty acid residues, and the third hydroxyl group of the glycerol moiety being bound to a phosphate which in turn is esterified with an alcohol moiety. Depending on the alcohol moiety, glycerophospholipids in general are divided into glycerophosphatidyl acids [PA], glycerophosphatidylcholines [phosphatidylcholines, PC], glycerophosphatidylethanolamines [PE], glycerophosphatidylglyceroles [PG], glycerophosphatidylinositoles [PI], glycerophosphatidylinositol bisphosphates (PIP2) and glycerophosphatidylinositol triphosphates [PIP3] and glycerophosphatidylserine (PS). The afore mentioned glycerophospholipids in turn are subdivided depending on whether their fatty acid residues are bound to the glycerol moiety by an ester (acyl) bond [a] or by an ether bond [e]. Two characters [aa, ea or ee] mean that two positions of the glycerol bear fatty acid residues, whereas only one character [a or e] stands for a so called lysoglycerophospholipid (e.g. lysophpsphatidylcholin) having only one fatty acid residue.

With the above definitions, e.g. the abbreviation "PC ae C42:1" stands for a glycerophosphatidylcholine having 42 carbon atoms in both of its fatty acid residues, i.e. the total number of carbons in both fatty acid side chains, and a single double bond in one of the fatty acid residues, i.e. the total number of double bonds in the fatty acid residues.

Yet another lipid class of interest is sphingomyelins and hydroxysphingomyelins [SMx:y; SM(OH)x:y, with x being the number of total carbons in both carbon chains and y being the total number of double bonds in both carbon chains].

In particular, the present invention provides a solution to the above mentioned problems in diagnosing stroke at an early stage, based on the application of a new technology in this context and on an unknown list of metabolic/metabolite compounds as diagnostic markers. Since metabolite concentration differences in biological fluids and tissues provide links to the various phenotypical responses, metabolites are suitable biomarker candidates.

The present invention allows for accurate, rapid, and sensitive prediction and diagnosis of stroke in subjects through a measurement of a plurality of metabolic biomarkers (metabolites) taken from a biological sample at a single point in time. This is accomplished by obtaining a biomarker panel at a single point in time from an individual, particularly an individual at risk of developing stroke in subject, having stroke in subject, or suspected of having stroke in subject, and comparing the biomarker profile from the individual to reference biomarker values or scores. The reference biomarker values may be obtained from a population of individuals (a "reference population") who are, for example, afflicted with stroke or who are suffering from stroke or a particular stage in the progression of stroke. If the biomarker panel values or score from the individual contains appropriately characteristic features of the biomarker values or scores from the reference population, then the individual is diagnosed as having a more likely chance of getting stroke in subject, as being afflicted with stroke or as being at the particular stage in the progression of stroke as the reference population.
Accordingly, the present invention provides, inter alia, methods of predicting the likelihood of an onset of stroke in an individual. The methods comprises obtaining a biomarker score at a single point in time from the individual and comparing the individual's biomarker profile to a reference biomarker profile. Comparison of the biomarker profiles can predict the onset of stroke in the individual preferably with a specificity of >90%. This method may be repeated again at any time prior to the onset of stroke in a subject.
The present invention further provides a method of determining the progression (i.e., the stage) of stroke in subject in an individual. This method comprises obtaining a biomarker profile composed of metabolite concentrations selected from table 1 at a single point in time from the individual and comparing the individual's biomarker profile to a reference biomarker score. Comparison of the biomarker scores can determine the progression of stroke in the individual with high specificity This method may also be repeated on the individual at any time.
Additionally, the present invention provides a method of diagnosing stroke in an individual having or suspected of having stroke. This method comprises obtaining a biomarker score at a single point in time from the individual and comparing the individual's biomarker score to a reference biomarker score. This method may also be repeated on the individual at any time.
The present invention further provides, inter alia, a method of determining the status of stroke in subject or diagnosing stroke in an individual comprising obtaining a biomarker score from a biological sample taken from the individual and comparing the individual's metabolite biomarker score to a reference bile acid biomarker score. A single such comparison is capable of classifying the individual as having membership in the reference population. Comparison of the biomarker scores determines the status or diagnoses stroke in the individual.
In yet another embodiment, the present invention provides, inter alia, a method of determining the status of stroke or diagnosing stroke in an individual. The method comprises comparing a measurable characteristic of more than one biomarker between a metabolite biomarker panel or biomarker score composed by (processed or unprocessed) values of this panel obtained from a biological sample from the individual and a biomarker score obtained from biological samples from a reference population. Based on this comparison, the individual is classified as belonging to or not belonging to the reference population. The comparison, therefore, determines the likelihood of stroke or diagnoses of such stroke in the individual. The biomarkers of the present invention are selected from the list of metabolites shown in Table 1.

**Table 1**

| **Metabolic Compound** | **Abbreviation** |
|---|---|
| Carnitine | C0 |
| Propionylcarnitine | C3 |
| Butyrylcarnitine | C4 |
| Valerylcarnitine | C5 |
| Tetradecanoylcarnitine | C14 |
| Octadecanoylcarnitine | C18 |
| Hydroxyvalerylcarnitine/ Methylmalonylcarnitine | C5-OH/ C3-DC-M |
| 4β-Hydroxycholesterol | 4b-OH-C |
| Lanosterol | |
| Desmosterol | |
| Arginine | Arg |
| Asparaginic acid | Asp |
| Tyrosine | Tyr |
| Ornithine | Orn |
| Threonine | Thr |
| Phenylalanine | Phe |
| Serine | Ser |
| Glutamine | Glu |
| Arachidonic acid | AA |
| Serotonin | |
| Phospatidylcholine acyl-alkyl C42:1 | PC ae C42:1 |
| Phospatidylcholine acyl-alkyl C40:1 | PC ae C40:1 |
| Phospatidylcholine acyl-alkyl C36:3 | PC ae C36:3 |
| Phospatidylcholine acyl-alkyl C36:4 | PC ae C36:4 |
| Phospatidylcholine acyl-alkyl C36:5 | PC ae C36:5 |
| Phospatidylcholine acyl-alkyl C38:4 | PC ae C38:4 |
| Phospatidylcholine acyl-alkyl C38:5 | PC ae C38:5 |
| Phospatidylcholine diacyl C42:1 | PC aa C42:1 |
| Phospatidylcholine diacyl C34:4 | PC aa C34:4 |
| Lysophospatidylcholine acyl C24:0 | lysoPC a C24:0 |
| Lysophospatidylcholine acyl C16:1 | lysoPC a C16:1 |
| Lysophospatidylcholine acyl C14:0 | lysoPC a C14:0 |
| Lysophospatidylcholine acyl C20:3 | lysoPC a C20:3 |
| Lysophospatidylcholine acyl C17:0 | lysoPC a C17:0 |
| Lysophospatidylcholine acyl C18:1 | lysoPC a C18:1 |
| Lysophospatidylcholine acyl C18:0 | lysoPC a C18:0 |
| Lysophospatidylcholine acyl C16:0 | lysoPC a C16:0 |
| Lysophospatidylcholine acyl C16:1 | lysoPC a C16:1 |
| Glycocholic acid | GCA |
| Glycochenodeoxycholic acid | GCDCA |
| Chenodeoxycholic acid | CDCA |
| Glycolithocholic acid | GLCA |
| Glycolithocholic acid sulfate | GLCAS |
| Taurocholic acid | TCA |
| Taurolithocholic acid | TLCA |
| Taurine-conjugated Taurochenodeoxycholic acid | TCDCA |
| Taurolithocholic acid sulfate | TLCAS |
| Taurodeoxycholic acid | TDCA |
| Docosahexaenoic acid | DHA |

wherein in acylcarnitines, phosphatidylcholines, and lysophosphatidylcholines, the number following "C" represents the number of total carbon atoms in the fatty acid and/or alkyl residue(s), and the number after the colon represents the number of total double bonds in the fatty acid residue and/or alkyl residue(s).

Biomarkers for diagnosing stroke (cerebrovascular accident) are characterized by consisting of a plurality of at least two metabolic compounds being specific for stroke, which are selected from the group consisting of the compounds of table 1.

However, within the scope of the present invention, any subset of combinations of the metabolites disclosed in table 1, beginning with 2 ,i.e. pairs of metabolites, to n-tuples with n being the maximum number of the metabolites contained in table 1 are possible and enabling embodiments of the present invention.

In this context, particularly preferred combinations of markers which are useful biomarkers for the present disclosure are the subsets (n-tuples) of combinations as listed in table 2:

**Table 2: Subsets 1-8 of Preferred Metabolites for Stroke Diagnosis**

| Subset No. | Metabolic Compounds |
|---|---|
| 1 | C5-OH (C3-DC-M),Tyr,Orn,GLCA,Thr,C3,Met,4b-OH-C,Phe,TLCA,C5,CO,Ser,PC ae C42:1, Serotonin, PC aa C42:1,CDCA,C4,Glu,lysoPC a C24:0 |
| 2 | C5-OH (C3-DC-M),Tyr,Orn,GLCA,Thr,C3,Met,4b-OH-C,Phe,TLCA |
| 3 | C5-OH (C3-DC-M),Tyr,Orn,GLCA,Thr |
| 4 | C5-OH (C3-DC-M),Tyr,Orn,GLCA |
| 5 | Tyr,Orn,GLCA,Thr |
| 6 | C5-OH (C3-DC-M),Tyr,Orn |
| 7 | C5-OH (C3-DC-M),Tyr |
| 8 | Orn,GLCA,Thr |

wherein in acylcarnitines, phosphatidylcholines, and lysophosphatidylcholines, the number following "C" represents the number of total carbon atoms in the fatty acid and/or alkyl residue, and the number after the colon represents the number of total double bonds in the fatty acid residue and/or alkyl residue.

A further preferred list of subsets of combinations of metabolites of table 1 is shown in the following table 3:

**Table 3: Subsets 9-17 of Preferred Metabolites for Stroke Diagnosis**

| Subset No | Metabolic Compounds |
|---|---|
| 9 | Asp,lysoPC a C16:1,C18,lysoPC a C14:0,lysoPC a C20:3,lysoPC a C17:0,C5-OH (C3-DC-M), lysoPC a C18:1,lysoPC a C18:0,GCA,TCDCA,GCDCA,lysoPC a C16:0,Lanosterol,GLCAS,TLCAS, TDCA,DHA,PC ae C36:3,PC aa C34:4 |
| 10 | Asp,lysoPC a C16:1,C18,lysoPC a C14:0,lysoPC a C20:3,lysoPC a C17:0,C5-OH (C3-DC-M), lysoPC a C18:1,lysoPC a C18:0,GCA |
| 11 | Asp,lysoPC a C16:1,C18,lysoPC a C14:0,lysoPC a C20:3 |
| 12 | Asp,lysoPC a C16:1,C18,lysoPC a C14:0 |
| 13 | Asp,lysoPC a C16:1,C18 |
| 14 | lysoPC a C16:1,C18,lysoPC a C14:0 |
| 15 | C18,lysoPC a C14:0,lysoPC a C20:3 |
| 16 | Asp,lysoPC a C16:1 |
| 17 | lysoPC a C16:1,C18 |

wherein in acylcarnitines, phosphatidylcholines, and lysophosphatidylcholines, the number following "C" represents the number of total carbon atoms in the fatty acid and/or alkyl residue, and the number after the colon represents the number of total double bonds in the fatty acid residue and/or alkyl residue.

**Table 4: Subsets 18-27 of Preferred Metabolites for Stroke, which are the subsets of metabolite combinations according to the present invention.**

| Subset No | Metabolic Compounds |
|---|---|
| 18 | C18,Lanosterol,Desmosterol,Asp,AA,lysoPC a C17:0,PC ae C36:4,PC ae C36:5,TDCA,TCDCA,TLCAS, DHA,GLCAS,Thr,Glu,TCA,Arg,lysoPC a C16:0,PC ae C38:4,PC ae C36:3,lysoPC a C18:0,PC ae C38:5, C14,PC ae C40:1,lysoPC a C16:1 |
| 19 | C18,Lanosterol,Desmosterol,Asp,AA,lysoPC a C17:0,PC ae C36:4,PC ae C36:5,TDCA,TCDCA,TLCAS,DHA ,GLCAS,Thr,Glu,TCA,Arg,lysoPC a C16:0,PC ae C38:4,PC ae C36:3 |
| 20 | C18,Lanosterol,Desmosterol,Asp,AA,PC ae C36:4,TDCA,TCDCA,TLCAS,DHA,GLCAS,Thr,Glu,TCA,Arg, lysoPC a C16:0,PC ae C36:3 |
| 21 | C18,Lanosterol,Desmosterol,Asp,AA,lysoPC a C17:0,PC ae C36:4,PC ae C36:5,TDCA,TCDCA |
| 22 | C18,Lanosterol,Desmosterol,Asp,AA |
| 23 | C18,Lanosterol,Desmosterol,Asp |
| 24 | C18,Lanosterol,Desmosterol |
| 25 | C18,Lanosterol |
| 26 | Lanosterol,Desmosterol,Asp |
| 27 | Desmosterol,Asp,AA |

wherein in acylcarnitines, phosphatidylcholines, and lysophosphatidylcholines, the number following "C" represents the number of total carbon atoms in the fatty acid and/or alkyl residue, and the number after the colon represents the number of total double bonds in the fatty acid residue and/or alkyl residue.
Although, each of the subsets of metabolites in accordance with tables 2 to 4 can be used in general for the diagnosis of stroke, it is preferred to use the subsets 1 to 8 (Table 2) in particular within 12h after clinical symptoms of stroke have occurred.
Subsets 9 to 17 (Table 3) are particularly useful within 48h of occurrence of clinical symptoms of stroke, and subsets 18 to 27 are preferred within 96h after clinical symptoms of stroke have occurred.
The suitability of the subset of metabolites of Tables 2 to 4 for diagnosing stroke is evident from the biostatistical data shown in Tables 5 to 7 below.

**Table 5: Biostatistical Data of Subsets 1 to 8 for stroke correlation within 12 h**

| Subset No. | AUC [%] | AUC CI95 [%] | Specificity [%] | Sensitivity [%] |
|---|---|---|---|---|
| 1 | 72.3 | 69.8-74.8 | 52.5 | 85.3 |
| 2 | 71 | 68.5-73.5 | 58.6 | 76.4 |
| 3 | 72.4 | 69.8-74.9 | 70.1 | 69.9 |
| 4 | 70.1 | 67.5-72.6 | 63.0 | 73.9 |
| 5 | 65.2 | 62.6-67.8 | 57.3 | 68.8 |
| 6 | 68.7 | 66.0-71.3 | 53.6 | 84.1 |
| 7 | 73.0 | 70.4-75.6 | 56.6 | 85.8 |
| 8 | 66.1 | 63.5-68.7 | 73.3 | 53.9 |

**Table 6: Biostatistical Data of Subsets 9 to 17 for stroke correlation within 48 h**

| Subset No. | AUC [%] | AUC CI95 [%] | Specificity [%] | Sensitivity [%] |
|---|---|---|---|---|
| 9 | 79.9 | 77.4-82.5 | 79.4 | 73.1 |
| 10 | 77.3 | 74.7-79.9 | 68.9 | 77.9 |
| 11 | 80.1 | 77.6-82.5 | 69.4 | 81.6 |
| 12 | 81.3 | 79.0-83.7 | 62.8 | 89.9 |
| 13 | 79.9 | 77.4-82.3 | 63.0 | 90.1 |
| 14 | 71.0 | 68.1-74.0 | 70.7 | 66.0 |
| 15 | 73.0 | 70.1-75.8 | 70.6 | 70.1 |
| 16 | 82.6 | 80.3-85.0 | 70.6 | 89.6 |
| 17 | 70.2 | 67.3-73.2 | 59.3 | 76.2 |

**Table 7: Biostatistical Data of Subsets 18 to 27 for stroke correlation within 96 h**

| Subset No. | AUC [%] | AUC CI95 [%] | Specificity [%] | Sensitivity [%] |
|---|---|---|---|---|
| 18 | 88.8 | 86.9-90.8 | 88.3 | 78.5 |
| 19 | 91.2 | 89.6-92.9 | 90.3 | 80.4 |
| 20 | 89.8 | 88.0-91.6 | 89.7 | 80.9 |
| 21 | 89.1 | 87.4-90.9 | 83.5 | 80.3 |
| 22 | 89.7 | 88.1-91.3 | 73.0 | 87.5 |
| 23 | 89.6 | 87.9-91.3 | 68.3 | 94.7 |
| 24 | 89.6 | 87.9-91.3 | 85.9 | 76.8 |
| 25 | 84.4 | 82.3-86.4 | 66.4 | 86.1 |
| 26 | 87.9 | 86.0-89.8 | 70.7 | 95.8 |
| 27 | 83.2 | 81.0-85.4 | 67.3 | 83.9 |

The present invention provides methods for diagnosing and/or predicting stroke in a subject. Such methods comprise the steps of: analyzing a biological sample from a subject to determine the levels of more than one biomarkers for stroke in the sample, where the one or more biomarkers are selected from Table 1 and comparing the levels of the biomarkers, as well as a composed value/score generated by subjecting the concentrations of individual biomarkers in the sample to a classification method such as affording an equation to process single concentration values - to obtain a separation between both (diseased and healthy) groups or comparing the level(s) of the one or more biomarkers in the sample to stroke in positive or stroke negative reference levels of the one or more biomarkers in order to determine whether the subject has had stroke.
The present invention provides a solution to the problem described above, and generally relates to the use of metabolomics data, generated by quantitation of metabolites by but not limited to mass spectrometry (MS), in particular MS-technologies such as MALDI, ESI, atmospheric pressure chemical ionization (APCI), and other methods, determination of metabolite concentrations by use of MS-technologies or alternative methods coupled to separation (LC-MS, GC-MS, CE-MS), subsequent feature selection and combination of features to classifiers including molecular data of at least two molecules.
The concentrations of the individual markers, analytes, metabolites thus are measured and compared to reference values or data combined and processed to scores, classifiers and compared to reference values thus indicating diseased states etc. with superior sensitivities and specificities compared to known procedures, clinical parameters and biomarkers.
Those skilled in the art will understand that for the quantitation of certain metabolites, also chemically modified metabolites may be used as one may get a better separation on the column material used prior to the MS-technologies.
Furthermore, in some embodiments, the present invention provides a method of diagnosing stroke in a subject and/or duration/severity comprising: detecting (the presence or absence of 2 or more, 5 or more, 10 or more, etc. metabolites measured together in a multiplex or panel format) stroke in subject specific metabolites in a sample (e.g., a tissue (e.g., biopsy) sample, a blood sample, a serum sample, or a urine sample) from a subject; and diagnosing stroke based on the presence of specific metabolites.
In a preferred embodiment of the invention, the method is characterized in that a deproteinization step and/or a separation step is inserted between steps a) and b), wherein said separation step is selected from the group consisting of liquid chromatography (LC), high performance liquid chromatography (HPLC), gas chromatography, liquid-liquid-extraction (LLE).

Said deproteinization step preferably is carried out by mixing said biological sample with organic solvents such as ethanol, methanol or acetonitrile.

In order to enhance sensitivity and/or volatility, e.g. for a better evaporation as used in mass spectrometry, the compounds can be derivatized as esters, amines or amides, wherein said derivatization includes: 2-Hydrazinopyridine (HP), 2-picolylamine (PA); Girard derivatization; oximation with hydroxylamine first and then silylation with hexamethyldisilazane and trifluoroacetic acid.

It is further preferred that said calibration step is carried out by
a) mathematically preprocessing said values in order to reduce technical errors being inherent to the measuring procedures used in accordance with the present invention, such as mass spectrometry.
b) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbor classifiers (K-NN), and applying said selected classifier algorithm to said preprocessed data of step a);
c) said classifier algorithms of step b) being trained on at least one training data set containing preprocessed data from subjects being divided into classes according to their stroke in subject-related pathophysiological, physiological, prognostic, or responder conditions , in order to select a classifier function to map said preprocessed data to said conditions;
d) applying said trained classifier algorithms of step c) to a preprocessed data set of a subject with unknown stroke in subject-related pathophysiological, physiological, prognostic, or responder condition, and using the trained classifier algorithms to predict the class label of said data set in order to predict the likelihood of an onset of stroke in a subject.

The step of mathematically preprocessing can be carried out e.g. by means of a statistical method on obtained raw data, particularly raw intensity data obtained by a measuring device, wherein said statistical method is selected from the group consisting of raw data obtained by mass spectrometry or mass spectrometry coupled to liquid or gas chromatography or capillary electrophoresis or by 2D gel electrophoresis, quantitative determination with RIA or determination of concentrations/amounts by quantitation of immunoblots; smoothing, baseline correction, peak picking, optionally, additional further data transformation such as taking the logarithm in order to carry out a stabilization of the variances.

Furthermore, for reasons of better accuracy of the prognostic results, a further step of feature selection may be inserted into said preprocessing step, in order to find a lower dimensional subset of features with the highest discriminatory power between classes; and/or
said feature selection is carried out by a filter and/or a wrapper approach;
and/or
wherein
said filter approach includes rankers and/or feature subset evaluation methods; and/or wherein said wrapper approach is applied, where a classifier is used to evaluate attribute subsets.

For the purpose of the present application, a pathophysiological condition, e.g. stroke, corresponds to the label "diseased" and said physiological condition corresponds to the label "healthy" or said pathophysiological condition corresponds to different labels of "grades of a disease", "subtypes of a disease", different values of a "score for a defined disease"; said prognostic condition corresponds to a label "good", "medium", "poor", or "therapeutically responding" or "therapeutically non-responding" or "therapeutically poor responding".

Typically, the method of the present invention is characterized in that said measuring step is carried out by high-throughput mass spectrometry.

It is preferred, that said stroke specific compounds are naturally occurring stroke specific metabolic compounds.

Furthermore, in the method according to the present invention, typically, said subject is a human being, and said biological sample is blood wherein raw data of metabolite concentrations are preprocessed using the log transformation;
wherein linear models are used to identify metabolites which are differentially present; wherein random forest (RF), K nearest neighbours (KNN), or linear discriminant analysis (LDA) is selected as suitable classifying algorithm, and is trained with preprocessed metabolite concentrations,
applying the obtained trained classifier to said preprocessed metabolite concentration data set of a subject under suspicion of having stroke in subject, and using said trained classifier to predict or diagnose stroke in subject

A further embodiment of the present invention is a use of a plurality of compounds being selected from the group of metabolites listed in table 1 for carrying out a method for predicting the likelihood of an onset of stroke and/or disorders related thereto in a patient.

A preferred method is one, wherein the biological sample is blood, plasma or serum.

Data classification is the categorization of data for its most effective and efficient use. Classifiers are typically deterministic functions that map a multi-dimensional vector of biological measurements to a binary (or n-ary) outcome variable that encodes the absence or existence of a clinically-relevant class, phenotype, distinct physiological state or distinct state of disease. To achieve this various classification methods such as, but not limited to, logistic regression, (diagonal) linear or quadratic discriminant analysis (LDA, QDA, DLDA, DQDA), perceptron, shrunken centroids regularized discriminant analysis (RDA), random forests (RF), neural networks (NN), hidden Markov models, generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbor classifiers (K-NN), fuzzy classifiers, bagging, boosting can be used.

Further aspects, advantages and embodiments of the present invention will become evident by the description of examples, from the experimental sections below and by means of the drawings.

As used herein, the term "stroke specific metabolite" refers to metabolites that are differentially present or differentially concentrated in brain-damaged organisms compared to healthy organisms.
A stroke -specific metabolite is preferably differentially present at a level that is statistically significant (e.g., an adjusted p-value less than 0.05 as determined using either Analysis of Variance, Welch's t-test or its non parametric equivalent versions). Exemplary stroke-specific metabolites are described in the detailed description and experimental sections below.
A biological sample may contain any biological material suitable for detecting the desired biomarkers, and may comprise cellular and/or non-cellular material from a subject. The sample can be isolated from any suitable biological tissue or fluid such as, for example, tissue, blood, blood plasma, urine. The biological sample according to the present application is at least one blood sample. A "reference level" of a metabolite means a level of the metabolite that is indicative of a particular disease state, phenotype, or lack thereof, as well as combinations of disease states, phenotypes, or lack thereof. A "positive" reference level of a metabolite means a level that is indicative of a particular disease state or phenotype. A "negative" reference level of a metabolite means a level that is indicative of a lack of a particular disease state or phenotype. For example, a stroke in subject -positive reference level" of a metabolite means a level of a metabolite that is indicative of a positive diagnosis of stroke in a subject, and a stroke-negative reference level" of a metabolite means a level of a metabolite that is indicative of a negative diagnosis of stroke in a subject. A "reference level" of a metabolite may be an absolute or relative amount or concentration of the metabolite, a presence or absence of the metabolite, a range of amount or concentration of the metabolite, a minimum and/or maximum amount or concentration of the metabolite, a mean amount or concentration of the metabolite, and/or a median amount or concentration of the metabolite; and, in addition, "reference levels" of combinations of metabolites may also be ratios of absolute or relative amounts or concentrations of two or more metabolites with respect to each other or a composed value / score obtained by classification.
As used herein, the term "processor" refers to a device that performs a set of steps according to a program (e.g., a digital computer). Processors, for example, include Central Processing Units ("CPUs"), electronic devices, or systems for receiving, transmitting, storing and/or manipulating data under programmed control.

As used herein, the term "memory device," or "computer memory" refers to any data storage device that is readable by a computer, including, but not limited to, random access memory, hard disks, magnetic (floppy) disks, compact discs, DVDs, magnetic tape, flash memory, and the like.
"Mass Spectrometry" (MS) is a technique for measuring and analyzing molecules that involves fragmenting a target molecule, then analyzing the fragments, based on their mass/charge ratios, to produce a mass spectrum that serves as a "molecular fingerprint". Determining the mass/charge ratio of an object is done through means of determining the wavelengths at which electromagnetic energy is absorbed by that object. There are several commonly used methods to determine the mass to charge ratio of an ion, some measuring the interaction of the ion trajectory with electromagnetic waves, others measuring the time an ion takes to travel a given distance, or a combination of both. The data from these fragment mass measurements can be searched against databases to obtain definitive identifications of target molecules. Mass spectrometry is also widely used in other areas of chemistry, like petrochemistry or pharmaceutical quality control, among many others.
As used here, the term "metabolic compound" or "metabolite" denotes organic compounds measured in a cell, an organism, a tissue or being present in body liquids and in extracts obtained from the aforementioned sources with a molecular weight typically below 1500 Dalton. Typical examples of metabolites/metabolic compounds are carnitine, acylcarnitines, bile acids, carbohydrates, fatty acids, lipids, phospholipids, sphingolipids and sphingophospholipids, amino acids, cholesterol, steroid hormones and oxidized sterols and other compounds such as collected in the Human Metabolite Database [Wishart DS et al., HMDB: the Human Metabolome Database. Nucleic Acids Res. 2007 Jan;35 (Database issue):D521-6 (see http://www.hmdb.ca/)] and other databases and literature. This includes any substance produced by metabolism or by a metabolic process and any substance involved in metabolism.

"Metabolomics" as understood within the scope of the present invention designates the comprehensive quantitative measurement of several (2-thousands) metabolites by, but not limited to, methods such as mass spectroscopy, coupling of liquid chromatography, gas chromatography and other separation methods with mass spectroscopy.

The term "separation" refers to separating a complex mixture into its component proteins or metabolites. Common laboratory separation techniques include gel electrophoresis and chromatography.

The term "capillary electrophoresis" refers to an automated analytical technique that separates molecules in a solution by applying voltage across buffer-filled capillaries. Capillary electrophoresis is generally used for separating ions, which move at different speeds when the voltage is applied, depending upon the size and charge of the ions. The solutes (ions) are seen as peaks as they pass through a detector and the area of each peak is proportional to the concentration of ions in the solute, which allows quantitative determinations of the ions.

The term "chromatography" refers to a physical method of separation in which the components to be separated are distributed between two phases, one of which is stationary (stationary phase) while the other (the mobile phase) moves in a definite direction. Chromatographic output data may be used for manipulation by the present invention.
An "ion" is a charged object formed by adding electrons to or removing electrons from an atom.
A "mass spectrum" is a plot of data produced by a mass spectrometer, typically containing m/z values on x-axis and intensity values on y-axis.
A "peak" is a point on a mass spectrum with a relatively high y-value.
The term "m/z" refers to the dimensionless quantity formed by dividing the mass number of an ion by its charge number. It has long been called the "mass-to-charge" ratio.
The term "metabolism" refers to the chemical changes that occur within the tissues of an organism, including "anabolism" and "catabolism". Anabolism refers to biosynthesis or the buildup of molecules and catabolism refers to the breakdown of molecules.
As used herein, the terms "detect", "detecting", or "detection" may describe either the general act of discovering or discerning or the specific observation of a detectably labelled composition.
As used herein, the term "clinical failure" refers to a negative outcome following stroke in treatment.

A biomarker in this context is a characteristic, comprising data of at least two metabolites that is measured and evaluated as an indicator of biologic processes, pathogenic processes, or responses to a therapeutic intervention associated with stroke or related to stroke in subject treatment. A combined biomarker as used here may be selected from at least two small molecules and metabolites.

### DETAILED DESCRIPTION OF THE INVENTION

The present invention relates to markers of stroke in a subject and its duration/severity. In particular embodiments, the present invention provides metabolites that are differentially present in stroke. Experiments conducted during the course of development of the embodiments of the present invention identified a series of metabolites as being differentially present in subjects with stroke in comparison to those without stroke.

### Diagnostic Applications

In some embodiments, the present invention provides methods and compositions for diagnosing stroke in a subject, including but not limited to, characterizing risk of stroke, stage of stroke in subject, duration and severity etc. based on the presence of stroke-specific metabolites or their derivatives, precursors, metabolites, etc. Exemplary diagnostic methods are described below.
Thus, for example, a method of diagnosing (or aiding in diagnosing) whether a subject has stroke comprises (1) detecting the presence or absence or a differential level of a plurality of metabolites being specific for stroke, such specific metabolites are selected from table 1 and b) diagnosing stroke in a subject based on the presence, absence or differential concentration level of the stroke-specific metabolites.

In some embodiments, a computer-based analysis program is used to translate the raw data generated by the detection assay (e.g., the presence, absence, or amount of a stroke-specific metabolite) into data of predictive value for a clinician. The clinician can access the predictive data using any suitable means. Thus, in some embodiments, the present invention provides the further benefit that the clinician, who is not likely to be trained in metabolite analysis, need not understand the raw data. The data is presented directly to the clinician in its most useful form. The clinician is then able to immediately utilize the information in order to optimize the care of the subject.
The present invention contemplates any method capable of receiving, processing, and transmitting the information to and from laboratories conducting the assays, information provides, medical personal, and subjects. The profile data is then prepared in a format suitable for interpretation by a treating clinician. For example, rather than providing raw data, the prepared format may represent a diagnosis or risk assessment (e.g., likelihood of stroke being present), along with recommendations for particular treatment options. The data may be displayed to the clinician by any suitable method. For example, in some embodiments, the profiling service generates a report that can be printed for the clinician (e.g., at the point of care) or displayed to the clinician on a computer monitor.
In some embodiments, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.
When the amounts or levels of a plurality of metabolites in the sample are determined, the amounts or levels may be compared to stroke metabolite-reference levels, such as - stroke -positive and/or stroke-negative reference levels to aid in diagnosing or to diagnose whether the subject has stroke. Levels of the plurality of metabolites in a sample corresponding to the stroke -positive reference levels (e.g., levels that are the same as the reference levels, substantially the same as the reference levels, above and/or below the minimum and/or maximum of the reference levels, and/or within the range of the reference levels) are indicative of a diagnosis of stroke in the subject.
In addition, levels of a plurality metabolites that are differentially present (especially at a concentration level that is statistically significant) in the sample as compared to stroke-negative reference levels are indicative of a diagnosis of stroke in the subject. Levels of the two or more metabolites that are differentially present (especially at a level that is statistically significant) in the sample as compared to stroke-positive reference levels are indicative of a diagnosis of no stroke in the subject.

The level(s) of a plurality of the metabolites may be compared to stroke -positive and/or stroke-negative reference levels using various techniques, including a simple comparison (e.g., a manual comparison) of the level(s) of the one or more metabolites in the biological sample to stroke -positive and/or stroke -negative reference levels. The level(s) of the one or more metabolites in the biological sample may also be compared to stroke and/or stroke -negative reference levels using one or more statistical analyses (e.g., t-test, Welch's t-test, Wilcoxon's rank sum test, random forests, linear discriminant analysis, k nearest neighbours).

Embodiments of the present invention provide for multiplex or panel assays that simultaneously detect a plurality (at least two) of the markers of the present invention depicted in table 1. For example, in some embodiments, panel or combination assays are provided that detected 3 or more, 4 or more, 5 or more, 6 or more, 7 or more, 8 or more, 9 or more, 10 or more, 5 or more markers in a single assay. In some embodiments, assays are automated or high throughput.

A preferred embodiment of the present invention is the use of markers listed in table 1 for diagnosis of stroke in subject and its duration/severity where said mammalian subject is a human being, said biological sample blood and/or blood cells.

### EXPERIMENTAL CONDITIONS

The following examples are provided in order to demonstrate and further illustrate certain preferred embodiments and aspects of the present invention and are not to be construed as limiting the scope thereof.
**Patients:** serum samples of patients with confirmed partial and territorial stroke and control patients (see table 8) were analyzed by quantitative LC-MS applying methods outlined below and several differentially concentrated metabolites were identified (table 1). Stroke was confirmed by classical clinical diagnosis and neurological outcome tests as well as by MRT. Control subjects were hospitalized patients with infections and a variety of other disorders except stroke.

### General Analytics:

Sample preparation and metabolomic analyses were performed at BIOCRATES life sciences AG, Innsbruck, Austria. We used a multi-parametric, highly robust, sensitive and high-throughput targeted metabolomic platform consisting of flow injection analysis (FIA)-MS/MS and LC-MS/MS methods for the simultaneous quantification of a broad range of endogenous intermediates namely from the panel disclosed in table1. All procedures (sample handling, analytics) were performed by co-workers blinded to the groups.

### Sample handling

### Plasma

Plasma samples were prepared by standard procedures and stored at (-75°C). To enable analysis of all samples simultaneously within one batch, samples were thawed on ice (1 h) on the day of analysis and centrifuged at 18000 g at 2°C for 5 min. All tubes were prepared with 0.001% BHT (butylated hydroxytoluene; Sigma-Aldrich, Vienna, Austria) to prevent autooxidation.

### Detailed Examples

### Mass spectroscopy

We used a multiparametric, highly robust, sensitive and high-throughput targeted metabolomic LC-MS/MS method for the simultaneous quantification of endogenous intermediates (amino acids, biogenic amines, acylcarnitines, sphingomyelins and glycerophospholipids, eicosanoides, oxysterols) in plasma samples enabling the determination of a broad range of analytes. All assays/ analytical methods are validated according the FDA guidance for bioanalytical methods for human plasma.

### Acylcarnitines, Amino acids, Sphingomyelins, Glycerophosphatidylcholines (FIA-MS/MS)

To determine the concentration of acylcarnitines, sphingomyelins and glycerophospholipids the AbsolutelDQ kit p150 (BIOCRATES Life Sciences AG, Innsbruck, Austria) was prepared as described in the manufacturer's protocol. In brief, 10 µl sample was added to the center of the filter on the upper 96-well kit plate, and the samples were dried using a nitrogen evaporator (VLM Laboratories, Leopoldshöhe, Germany). The metabolites were extracted using 300 µl of a 5 mM ammonium acetate solution in methanol. The extracts were obtained by centrifugation into the lower 96-deep well plate followed by a dilution step with 600 µl of kit MS running solvent. Mass spectrometric analysis was performed on an API 4000 Q Trap® tandem mass spectrometry instrument (Applied Biosystems/MDS Analytical Technologies, Darmstadt, Germany) equipped with a Turbo V™ electrospray ionization (ESI) source using the analysis acquisition method as provided in the AbsolutelDQ kit. The standard FIA-MS/MS method was applied for all measurements with two subsequent 20 µl injections (one for positive and one for negative mode analysis). Multiple reaction monitoring (MRM) detection was used for quantification applying the spectra parsing algorithm integrated into the MetlQ software (BIOCRATES Life Sciences AG, Innsbruck, Austria).

### Amino acids, Biogenic amines (LC-MS/MS)

Amino acids and biogenic amines were quantitatively analyzed by reversed phase LC-MS/MS to obtain chromatographic separation of isobaric (same MRM ion pairs) metabolites for individual quantitation performed by external calibration and by use of internal standards. 10 µl sample volume is required for the analysis using the following sample preparation procedure. Samples were added on filter spots placed in a 96-solvinert well plate (internal standards were placed and dried down under nitrogen before), fixed above a 96-deep well plate (capture plate). 20 µl of 5 % phenyl-isothiocyanate derivatization reagent was added. The derivatized samples were extracted after incubation by aqueous methanol into the capture plate. 10 µl sample extracts were analyzed by LC-ESI-MS/MS in positive MRM detection mode with a API 4000 Q Trap® tandem mass spectrometry instrument (Applied Biosystems/MDS Analytical Technologies, Darmstadt, Germany). The analyzed individual metabolite concentrations (Analyst 1.4.2 software, Applied Biosystems) were exported for comprehensive statistical analysis.

### Energy metabolism (Organic Acids) (LC-MS/MS)

For the quantitative analysis of energy metabolism intermediates (glycolysis, citrate cycle, pentose phosphate pathway, urea cycle) hydrophilic interaction liquid chromatography HILIC-ESI-MS/MS method in highly selective negative MRM detection mode was used. The MRM detection was performed using API 4000 QTRAP tandem mass spectrometry instrument (Applied Biosystems/MDS Analytical Technologies). 20 µL sample volume was protein precipitated and extracted simultaneously with aqueous methanol in a 96-well plate format. Internal standards (ratio external to internal standard)and external calibration were used for highly accurate quantitation. Data were quantified with Analyst 1.4.2 software (applied Biosystems) and finally exported for statistical analysis.

### Eicosanoids (Prostanoids, oxidized fatty acids) (LC-MS/MS)

Prostanoids - a term summarizing prostaglandins (PG), thromboxanes (TX) and prostacylines - and oxidised fatty acid metabolites were analyzed in plasma extracts by LC-MS/MS (Unterwurzacher at al. Clin Chem Lab Med 46 (11) (2008), 1589-1597) with an API5500 QTrap® tandem mass spectrometry instrument (Applied Biosystems/MDS Analytical Technologies) in negative MRM detection mode. In brief, filter spots in a 96 well plate were spiked with internal standard; 20 µL of plasma were added and extracted with aqueous methanol, the individual extracts then were analysed. Data of prostanoids and oxidized fatty acids were quantified with Analyst 1.5 software (Applied Biosystems) and finally exported for statistical analysis.

### Oxysterols (LC-MS/MS)

Oxysterols are determined after extraction and saponification by HPLC-Tandem mass spectrometer (HPLC-API-MS/MS) in positive detection mode using Multiple Reaction Mode (MRM). Samples (20 µL), calibrators and internal standard mixture were placed into a capture plate and were protein precipitated in the first step by means of addition of 200 µL acetonitrile and centrifugation. 180 µL of the appropriate supernatants were transferred on a new filter plate with 7 mm filter spots, dried down, hydrolysed with 0.35 M KOH in 95 % Ethanol and after washing steps extracted with 100 µL aqueous MeOH. An aliquot of the extracted sample is injected onto the HPLC-MS/MS system. Chromatographic separation and detection is performed by using a Zorbax Eclipse XDB C18, 150 x 2.0 mm, 3.5 µm HPLC-Column at a flow rate of 0.3 mL/min followed by electrospray ionization on the API5000 QTRAP tandem mass spectrometry instrument (Applied Biosystems/MDS Analytical Technologies). For the quantitation the Analyst 1.5 software from Applied Biosystems was used and the metabolite concentrations were exported for statistical analysis.

### Experimental Setup

A set of 74 human samples obtained from 28 patients 12 hours after beginning of clinical stay due to symptoms and/or suspicion of stroke event have been measured. For a subset of 15 patients, a time series of 3 additional time points (12h, 48h and 96h) has been sampled. The samples are described as follows:
L = left, R = right, M = media, A = anterior, P = posterior, T = partial infarct, K = complete infarct.

**Table 8: Patient Group**

| | 2D-Sample-ID | Patient | Sample Timepoint | Age | Gender | Height [cm] |
|---|---|---|---|---|---|---|
| 1910890 | 41505103 | P001 | 12h | 86 | F | 158 |
| 1910925 | 41504331 | P001 | 24h | 86 | F | 158 |
| 1910929 | 41505601 | P001 | 48h | 86 | F | 158 |
| 1910878 | 41505567 | P001 | 96h | 86 | F | 158 |
| 1910923 | 41504839 | P002 | 12h | 73 | F | 164 |
| 1910928 | 41505564 | P002 | 24h | 73 | F | 164 |
| 1910930 | 41505512 | P002 | 48h | 73 | F | 164 |
| 1910879 | 41504817 | P002 | 96h | 73 | F | 164 |
| 1910926 | 41504799 | P003 | 12h | 82 | F | 170 |
| 1910883 | 41504309 | P004 | 12h | 73 | F | 165 |
| 1910884 | 41505099 | P004 | 24h | 73 | F | 165 |
| 1910888 | 41503671 | P004 | 48h | 73 | F | 165 |
| 1910887 | 41503670 | P004 | 96h | 73 | F | 165 |
| 1910885 | 41503654 | P005 | 12h | 74 | F | 170 |
| 1910852 | 41504899 | P005 | 24h | 74 | F | 170 |
| 1910855 | 41499325 | P005 | 48h | 74 | F | 170 |
| 1910847 | 41501581 | P005 | 96h | 74 | F | 170 |
| 1910858 | 41499216 | P007 | 12h | 63 | M | 185 |
| 1910895 | 41503942 | P007 | 24h | 63 | M | 185 |
| 1910899 | 41500406 | P010 | 12h | 73 | M | 175 |
| 1910932 | 41500097 | P010 | 24h | 73 | M | 175 |
| 1910935 | 41502665 | P010 | 48h | 73 | M | 175 |
| 1910943 | 41512313 | P010 | 96h | 73 | M | 175 |
| 1910864 | 41512497 | P013 | 12h | 68 | M | 178 |
| 1917510 | 41513591 | P015 | 12h | 66 | M | 180 |
| 1917522 | 41499607 | P016 | 12h | 70 | M | 176 |
| 1917512 | 41518650 | P017 | 12h | 65 | F | 170 |
| 1917546 | 41515164 | P022 | 12h | 68 | M | 165 |
| 1917542 | 41515121 | P022 | 24h | 68 | M | 165 |
| 1917548 | 41514842 | P022 | 48h | 68 | M | 165 |
| 1917527 | 41514617 | P022 | 96h | 68 | M | 165 |
| 1917573 | 41518222 | P024 | 12h | 87 | F | 155 |
| 1921912 | C11 | P025 | 12h | 71 | F | 170 |
| 1921913 | 41517540 | P025 | 24h | 71 | F | 170 |
| 1921916 | 41517565 | P025 | 48h | 71 | F | 170 |
| 1921920 | 41517593 | P025 | 96h | 71 | F | 170 |
| 1921914 | 41512707 | P026 | 12h | 84 | F | 165 |
| 1921915 | 41512697 | P026 | 24h | 84 | F | 165 |
| 1921923 | 41515138 | P026 | 48h | 84 | F | 165 |
| 1917571 | 41518479 | P026 | 96h | 84 | F | 165 |
| 1921919 | 41518520 | P027 | 12h | 37 | M | 175 |
| 1921905 | 41515124 | P027 | 24h | 37 | M | 175 |
| 1921924 | 41518267 | P027 | 48h | 37 | M | 175 |
| 1921946 | 41515066 | P027 | 96h | 37 | M | 175 |
| 1917572 | 41515093 | P028 | 12h | 63 | M | 172 |
| 1921906 | 41515040 | P028 | 24h | 63 | M | 172 |
| 1921903 | 41518126 | P028 | 48h | 63 | M | 172 |
| 1921902 | 41518171 | P028 | 96h | 63 | M | 172 |
| 1921904 | 41518157 | P029 | 12h | 65 | M | 175 |
| 1921907 | 41518120 | P029 | 24h | 65 | M | 175 |
| 1921908 | 65128237 | P029 | 48h | 65 | M | 175 |
| 1921942 | 41500579 | P029 | 96h | 65 | M | 175 |
| 1921939 | 41500560 | P030 | 12h | 75 | F | 156 |
| 1916776 | 65130883 | P034 | 12h | 74 | M | 167 |
| 1916717 | 65130824 | P034 | 24h | 74 | M | 167 |
| 1914801 | 65130633 | P034 | 48h | 74 | M | 167 |
| 1914802 | 65131573 | P034 | 96h | 74 | M | 167 |
| 1916719 | 65131371 | P035 | 12h | 65 | F | 168 |
| 1916721 | 65130716 | P035 | 24h | 65 | F | 168 |
| 1916722 | 41500084 | P035 | 48h | 65 | F | 168 |
| 1916781 | 65131515 | P035 | 96h | 65 | F | 168 |
| 1916780 | 41517738 | P036 | 12h | 71 | M | 172 |
| 1921950 | 65131410 | P037 | 12h | 46 | F | 175 |
| 1916798 | 41516879 | P050 | 12h | 89 | M | 167 |
| 1916797 | 41516873 | P050 | 24h | 89 | M | 167 |
| 1916796 | 41515223 | P050 | 48h | 89 | M | 167 |
| 1916789 | 65135199 | P050 | 96h | 89 | M | 167 |
| 1916742 | 65135392 | P051 | 12h | 88 | F | 165 |
| 1916740 | 65131417 | P055 | 12h | 76 | M | 176 |
| 1916744 | 65135505 | P055 | 24h | 76 | M | 176 |
| 1916745 | 65131465 | P055 | 48h | 76 | M | 176 |
| 1916748 | 34628278 | P055 | 96h | 76 | M | 176 |
| 1931691 | 76918330 | P063 | 12h | 75 | F | 170 |
| 1916702 | 76938127 | P064 | 12h | 72 | F | 175 |

**Table 9: Stroke Locations in Patient Group**

| | Weight [kg] | Schlaganfalllokalisation | NIHSS | Comment |
|---|---|---|---|---|
| 1910890 | 60 | LMT | 15 | |
| 1910925 | 60 | LMT | 15 | |
| 1910929 | 60 | LMT | 15 | |
| | | | | |
| 1921903 | 75 | LMT | 19 | |
| 1921902 | 75 | LMT | 19 | |
| 1921904 | 80 | LMT | 1 | |
| 1921907 | 80 | LMT | 1 | |
| 1921908 | 80 | LMT | 1 | |
| 1921942 | 80 | LMT | 1 | |
| 1921939 | 70 | LMT | 21 | |
| 1916776 | 80 | RMK | 15 | |
| 1916717 | 80 | RMK | 15 | |
| 1914801 | 80 | RMK | 15 | |
| 1914802 | 80 | RMK | 15 | |
| 1916719 | 95 | RMT | 14 | |
| 1916721 | 95 | RMT | 14 | |
| 1916722 | 95 | RMT | 14 | |
| 1916781 | 95 | RMT | 14 | |
| 1916780 | 69 | LMK | 25 | |
| 1921950 | 75 | RMK | 16 | |
| 1916798 | 70 | RMT | 11 | |
| 1916797 | 70 | RMT | 11 | |
| 1916796 | 70 | RMT | 11 | |
| 1916789 | 70 | RMT | 11 | |
| 1916742 | 65 | LMT | 33 | |
| 1916740 | 82 | RAMT | 13 | |
| 1916744 | 82 | RAMT | 13 | |
| 1916745 | 82 | RAMT | 13 | |
| 1916748 | 82 | RAMT | 13 | |
| 1931691 | 83 | RMT | 12 | |
| 1916702 | 75 | LMT | 19 | |

| The samples can be summarized to the following analytically relevant groups: | | | | |
|---|---|---|---|---|
| | Overall | F | M | |
| 12 hours | 28 | 15 | 13 | |
| 24 hours | 16 | 7 | 9 | |
| 48 hours | 15 | 7 | 8 | |
| 96 hours | 15 | 7 | 8 | |
| All | 74 | 36 | 38 | |
| 153853 | 600256 | | Kontrolle | |
| 153941 | 622233 | | Kontrolle | |
| 153968 | 603214 | | Kontrolle | |
| 157194 | 613180 | | Kontrolle | |
| 147794 | 109041984 | | Kontrolle | |
| 147833 | 109104239 | | Kontrolle | |
| 147851 | 279315 | | Kontrolle | |
| 150758 | 612934 | | Kontrolle | |
| 150792 | 281538 | | Kontrolle | |
| 150859 | 610376 | | Kontrolle | |
| 150915 | 604611 | | Kontrolle | |
| 153819 | 619489 | | Kontrolle | |
| 153822 | 324648 | | Kontrolle | |
| 153898 | 625705 | | Kontrolle | |
| 153954 | 640435 | | Kontrolle | |
| 157584 | 644784 | | Kontrolle | |

| | Sample Identification | | Sample Description | |
|---|---|---|---|---|
| 147816 | 660120 | | Kontrolle | |
| 147878 | 290261 | | Kontrolle | |
| 150761 | 606519 | | Kontrolle | |
| 150775 | 602424 | | Kontrolle | |
| 150828 | 277584 | | Kontrolle | |
| 150845 | 276279 | | Kontrolle | |
| 150876 | 100507031 | | Kontrolle | |
| 150901 | 619600 | | Kontrolle | |
| 153783 | 628011 | | Kontrolle | |

Data preparation: The statistical computing environment R [R Development Core Team, 2010] was used to perform statistical analysis. All concentrations have been log2-transformed to obtain normally distributed analyte measurements and to stabilize variance. Metabolites with more than 50% missing values were removed from analysis.

### Statistical Analysis

All statistical calculations have been performed using the statistics software R (R: A Language and Environment for Statistical Computing, R Development Core Team, R Foundation for Statistical Computing, Vienna, Austria, 2010, ISBN 3-900051-07-0).
All analytes that were detected in at least 15% of the samples were selected for further analyses. The metabolic data is left censored due to thresholding of the mass spectrometer data resulting in non detected peak/signals. By a combination of metabolic pathway dynamism, complex sample molecular interaction and overall efficiency of the analytical protocol, replacement of missing data by means of a multivariate algorithm is preferred to a naive imputation by a pre-specified value like for instance zero. Hence, missing metabolite concentrations are replaced by the average value of the 6 closest samples to the one where the measurement is missing (impute: Imputation for microarray data, Hastie T., Tibshirani R., Narasimhan B. and Chu G., R package version 1.14.0). At the exception of fold change (FC) determination, all statistical analyses are performed on preprocessed - that is, log transformed - data. The log-transformation is used to stabilize variance and to transform to Gaussian distribution - at least approximately.
The ImFit function in the package limma (limma: linear models for microarray data, Smyth G.K. In: Bioinformatics and Computational Biology Solutions using R and Bioconductor, Springer, New York, pp 397-420, R package version 2.16.5) is used to compute the moderated statistics for pair wise comparisons between measurements from control samples and samples with stroke in subject. Resulting p values are adjusted by the method described in Benjamini and Hochberg (Benjamini Y. and Hochberg Y., Controlling the false discovery rate: a practical and powerful approach to multiple testing, Journal of the Royal Statistical Society Series B, 1995, 57, 289-300) leading to so-called q values. Sensitivity/specificity properties of a classifier comprising one analyte or a combination of analytes are summarised in terms of Area Under the Receiver Operating Characteristic Curve (AUC). The function colAUC (caTools: Tools: moving window statistics, GIF, Base64, ROC AUC, etc., Tuszynski J., 2008, R package version 1.9) is used to compute AUC values.
A higher reproducibility of the results can be achieved by combining criteria like log2-FC, p resp. q value and AUC.

Logistic regression has been applied to classify experimental groups based on combinations of biomarkers. The method is implemented in the R add-on package glmnet (version 1.7, 2011-05-29) and is based on regression shrinkage and feature selection by elastic net penalties (Friedman J., Hastie T., Tibshirani R. 2010. J Stat Software, 33(1), 1-22). In case of feature selection, grid search of the α-value based on leave-one-out cross-validation is processed in a range of α=[0.05,0.98]. For a fixed set of biomarkers the α-value is set to α=0 and only λ is grid-searched across 100 standard values. The classification power is assessed by repeated model building and predictions based on 100-fold bootstrap resampling. ROC curves are calculated with the R-package ₚROC (Robin X., Turck N., Hainard A. and others. 2011. BMC Bioinformatics, 12, 77) and 95% confidence intervals (Cl₉₅) are calculated based on 100-fold bootstrap resampling.

### Analysis strategy:

- Investigation of data integrity (correlation, plate effects).
- Univariate regression analysis to detect trends of metabolic change.
- T-tests for differences of stroke patients metabolites vs control patients metabolites
- ANOVA of differences in timepoints after stroke.

Trends of Stroke Recovery as linear Functions
Time series data is available for the following patients: "P001" "P002" "P004" "P005" "P010" "P022" "P025" "P026" "P027" "P028", "P029" "P034" "P035" "P050" "P055"
For these patients linear regression models of logarithmically transformed concentration values have been calculated. The applied linear model correlates the sampling time as explanatory variable with the the metabolite concentration as dependent variable. Linearity of the trend has been asured by a log-transformation. Only metabolites with a significant trend (slope significantly differs from zero with adjusted p-values below 0.05) have been considered.
Regression analysis is based due to the sampling for the first time point (12 hours) on all samples and for other time points on 16 or 15 patients (see samples overview), respectively.

## Claims

1. A method for diagnosing stroke (cerebrovascular accident) in patients within 96 h after occurrence of clinical symptoms,
**characterized by**
quantitatively detecting in vitro in at least one blood sample of a patient a plurality of metabolic compounds being specific for stroke, and having a molecular weight of less than 1500 Dalton comprising the steps of:
a) selecting said plurality of metabolic compounds comprising at least one of the sets of metabolites selected from the group consisting of:
| Set No | Metabolic Compounds |
|---|---|
| 18 | Octadecanoylcarnitine, Lanosterol, Desmosterol, Asp, Arachidonic acid, Lysophospatidylcholine acyl C17:0, Phospatidylcholine acyl-alkyl C36:4, Phospatidylcholine acyl-alkyl C36:5, Taurodeoxycholic acid, Taurine-conjugated taurochenodeoxycholic acid, Taurolithocholic acid sulfate, Docosahexaenoic acid, Glycolithocholic acid sulfate, Thr, Glu, Taurocholic acid, Arg, Lysophospatidylcholine acyl C16:0, Phospatidylcholine acyl-alkyl C38:4, Phospatidylcholine acyl-alkyl C36:3, Lysophospatidylcholine acyl C18:0, Phospatidylcholine acyl-alkyl C38:5, Tetradecanoylcarnitine, Phospatidylcholine acyl-alkyl C40:1, Lysophospatidylcholine acyl C16:1 |
| 19 | Octadecanoylcarnitine, Lanosterol, Desmosterol, Asp, Arachidonic acid, Lysophospatidylcholine acyl C17:0, Phospatidylcholine acyl-alkyl C36:4, Phospatidylcholine acyl-alkyl C36:5, Taurodeoxycholic acid, Taurine-conjugated taurochenodeoxycholic acid, Taurolithocholic acid sulfate, Docosahexaenoic acid, Glycolithocholic acid sulfate, Thr, Glu, Taurocholic acid, Arg, Lysophospatidylcholine acyl C16:0, Phospatidylcholine acyl-alkyl C38:4, Phospatidylcholine acyl-alkyl C36:3 |
| 20 | Octadecanoylcarnitine, Lanosterol, Desmosterol, Asp, Arachidonic acid, Phospatidylcholine acyl-alkyl C36:4, Taurodeoxycholic acid, Taurine-conjugated taurochenodeoxycholic acid, Taurolithocholic acid sulfate, Docosahexaenoic acid, Glycolithocholic acid sulfate, Thr, Glu, Taurocholic acid, Arg, Lysophospatidylcholine acyl C16:0, Phospatidylcholine acyl-alkyl C36:3 |
| 21 | Octadecanoylcarnitine, Lanosterol, Desmosterol, Asp, Arachidonic acid, Lysophospatidylcholine acyl C17:0, Phospatidylcholine acyl-alkyl C36:4, Phospatidylcholine acyl-alkyl C36:5, Taurodeoxycholic acid, Taurine-conjugated taurochenodeoxycholic acid |
| 22 | Octadecanoylcarnitine, Lanosterol, Desmosterol, Asp, Arachidonic acid |
| 23 | Octadecanoylcarnitine, Lanosterol, Desmosterol, Asp |
| 24 | Octadecanoylcarnitine, Lanosterol, Desmosterol |
| 25 | Octadecanoylcarnitine, Lanosterol |
| 26 | Lanosterol, Desmosterol, Asp |
| 27 | Desmosterol, Asp, Arachidonic acid |
wherein in acylcarnitines, phosphatidylcholines, and lysophosphatidylcholines, the number following "C" represents the number of total carbon atoms in the fatty acid and/or alkyl residue(s), and the number after the colon represents the number of total double bonds in the fatty acid residue and/or alkyl residue(s);
b) measuring at least one of the parameters selected from the group consisting of: presence, concentration, level or amount of each specific compound of said plurality of compounds in said sample; obtaining a qualitative and/or quantitative molecular pattern from the entirety of the obtained values from said parameter measurements; and storing said obtained set of values in a database;
c) calibrating said values by comparing clinically confirmed stroke-positive and/or clinically confirmed stroke-negative reference parameters; and
d) comparing said measured values in the sample with the calibrated values, in order to assess whether the patient is stroke-positive or stroke-negative.

2. Method according to claim 1, wherein stroke comprises ischemic stroke and hemorrhagic stroke.

3. Method according to claim 1 or 2, wherein said quantitative detection comprises establishing of a metabolomics profile which is achieved by a quantitative metabolomics profile analysis method comprising the generation of intensity data for the quantitation of said compounds by mass spectrometry (MS), in particular, by high-throughput mass spectrometry, preferably by MS-technologies such as Matrix Assisted Laser Desorption/lonisation (MALDI), Electro Spray Ionization (ESI), Atmospheric Pressure Chemical Ionization (APCI), ¹H-, ¹³C- and/or ³¹P- Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS).

4. Method according to anyone of claims 1 to 3, wherein intensity data of said metabolomics profile are normalized with a set of endogenous housekeeper metabolites by relating detected intensities of the selected target metabolites being specific for stroke to detected intensities of said housekeeper metabolites.

5. Method according to claim 4, wherein said housekeeper metabolites are selected from the group consisting of such metabolites which show stability in accordance with statistical stability measures being selected from the group consisting of coefficient of variation (CV) of raw intensity data, standard deviation (SD) of logarithmic intensity data, stability measure (M) of geNorm - algorithm or stability measure value (rho) of NormFinder-algorithm.

6. Method according to anyone of claims 1 to 5, wherein a panel of reference target compounds or derivatives thereof is established by:
a) mathematically preprocessing intensity values obtained for generating the metabolomics profiles in order to reduce technical errors being inherent to the measuring procedures used to generate the metabolomics profiles;
b) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbour classifiers (K-NN), fuzzy classifiers, bagging, boosting, and naïve Bayes; and applying said selected classifier algorithm to said preprocessed data of step a);
c) said classifier algorithms of step b) being trained on at least one training data set containing preprocessed data from subjects being divided into classes according to their likelihood to develop an inflammation-related brain injury, in order to select a classifier function to map said preprocessed data to said likelihood;
d) applying said trained classifier algorithms of step c) to a preprocessed data set of a subject with unknown likelihood of stroke, and using the trained classifier algorithms to predict the class label of said data set in order to predict the likelihood for a subject to suffer from stroke.

7. Method according to anyone of claims 1 to 6, wherein said predictive target metabolites for easier and/or more sensitive detection are detected by means of chemically modified derivatives thereof, such as phenyisothiocyanates for amino acids.

8. Use of a plurality of naturally occurring metabolic compounds for diagnosing stroke from a patient's blood sample in vitro within 96 h after occurrence of clinical symptoms, wherein said compounds have a molecular weight of less than 1500 Dalton, and wherein said plurality of the compounds comprises at least one of the sets of metabolites selected from the group consisting of:
| Set No | Metabolic Compounds |
|---|---|
| 18 | Octadecanoylcarnitine, Lanosterol, Desmosterol, Asp, Arachidonic acid, Lysophospatidylcholine acyl C17:0, Phospatidylcholine acyl-alkyl C36:4, Phospatidylcholine acyl-alkyl C36:5, Taurodeoxycholic acid, Taurine-conjugated taurochenodeoxycholic acid, Taurolithocholic acid sulfate, Docosahexaenoic acid, Glycolithocholic acid sulfate, Thr, Glu, Taurocholic acid, Arg, Lysophospatidylcholine acyl C16:0, Phospatidylcholine acyl-alkyl C38:4, Phospatidylcholine acyl-alkyl C36:3, Lysophospatidylcholine acyl C18:0, Phospatidylcholine acyl-alkyl C38:5, Tetradecanoylcarnitine, Phospatidylcholine acyl-alkyl C40:1, Lysophospatidylcholine acyl C16:1 |
| 19 | Octadecanoylcarnitine, Lanosterol, Desmosterol, Asp, Arachidonic acid, Lysophospatidylcholine acyl C17:0, Phospatidylcholine acyl-alkyl C36:4, Phospatidylcholine acyl-alkyl C36:5, Taurodeoxycholic acid, Taurine-conjugated taurochenodeoxycholic acid, Taurolithocholic acid sulfate, Docosahexaenoic acid, Glycolithocholic acid sulfate, Thr, Glu, Taurocholic acid, Arg, Lysophospatidylcholine acyl C16:0, Phospatidylcholine acyl-alkyl C38:4, Phospatidylcholine acyl-alkyl C36:3 |
| 20 | Octadecanoylcarnitine, Lanosterol, Desmosterol, Asp, Arachidonic acid, Phospatidylcholine acyl-alkyl C36:4, Taurodeoxycholic acid, Taurine-conjugated taurochenodeoxycholic acid, Taurolithocholic acid sulfate, Docosahexaenoic acid, Glycolithocholic acid sulfate, Thr, Glu, Taurocholic acid, Arg, Lysophospatidylcholine acyl C16:0, Phospatidylcholine acyl-alkyl C36:3 |
| 21 | Octadecanoylcarnitine, Lanosterol, Desmosterol, Asp, Arachidonic acid, Lysophospatidylcholine acyl C17:0, Phospatidylcholine acyl-alkyl C36:4, Phospatidylcholine acyl-alkyl C36:5, Taurodeoxycholic acid, Taurine-conjugated taurochenodeoxycholic acid |
| 22 | Octadecanoylcarnitine, Lanosterol, Desmosterol, Asp, Arachidonic acid |
| 23 | Octadecanoylcarnitine, Lanosterol, Desmosterol, Asp |
| 24 | Octadecanoylcarnitine, Lanosterol, Desmosterol |
| 25 | Octadecanoylcarnitine, Lanosterol |
| 26 | Lanosterol, Desmosterol, Asp |
| 27 | Desmosterol, Asp, Arachidonic acid |
wherein in acylcarnitines, phosphatidylcholines, and lysophosphatidylcholines, the number following "C" represents the number of total carbon atoms in the fatty acid and/or alkyl residue, and the number after the colon represents the number of total double bonds in the fatty acid residue and/or alkyl residue.

## Patentansprüche

1. Verfahren zum Diagnostizieren eines Schlaganfalls (cerebrovaskulärer Anfall) bei Patienten innerhalb von 96 h nach Auftreten klinischer Symptome,
**gekennzeichnet durch**
quantitative in vitro Erfassung in wenigstens einer Blutprobe eines Patienten einer Mehrzahl von Metabolitenverbindungen, welche für einen Schlaganfall spezifisch sind und welche ein Molekulargewicht von weniger als 1500 Dalton aufweisen, die folgenden Schritte umfassend:
a) Auswählen der Mehrzahl der Metabolitenverbindungen, umfassend wenigstens einen der Metabolitensets, welche ausgewählt sind aus der Gruppe, bestehend aus:
| Set Nr | Metabolitenverbindungen |
|---|---|
| 18 | Octadecanoylcarnitin, Lanosterol, Desmosterol, Asp, Arachidonsäure, Lysophospatidylcholinacyl C17:0, Phospatidylcholinacylalkyl C36:4, Phospatidylcholinacylalkyl C36:5, Taurodeoxycholsäure, Taurin-konjugierte Taurochenodeoxycholsäure, Taurolithocholsäuresulfat, Docosahexaensäure, Glycolithocholsäuresulfat, Thr, Glu, Taurocholsäure, Arg, Lysophospatidylcholinacyl C16:0, Phospatidylcholinacylalkyl C38:4, Phospatidylcholinacylalkyl C36:3, Lysophospatidylcholinacyl C18:0, Phospatidylcholinacylalkyl C38:5, Tetradecanoylcarnitin, Phospatidylcholinacylalkyl C40:1, Lysophospatidylcholinacyl C16:1 |
| 19 | Octadecanoylcarnitin, Lanosterol, Desmosterol, Asp, Arachidonsäure, Lysophospatidylcholinacyl C17:0, Phospatidylcholinacylalkyl C36:4, Phospatidylcholinacylalkyl C36:5, Taurodeoxycholsäure, Taurin-konjugierte Taurochenodeoxycholsäure, Taurolithocholsäuresulfat, Docosahexaensäure, Glycolithocholsäuresulfat, Thr, Glu, Taurocholsäure, Arg, Lysophospatidylcholinacyl C16:0, Phospatidylcholinacylalkyl C38:4, Phospatidylcholinacylalkyl C36:3 |
| 20 | Octadecanoylcarnitin, Lanosterol, Desmosterol, Asp, Arachidonsäure, Phospatidylcholinacylalkyl C36:4, Taurodeoxycholsäure, Taurin-konjugierte Taurochenodeoxycholsäure, Taurolithocholsäuresulfat, Docosahexaensäure, Glycolithocholsäuresulfat, Thr, Glu, Taurocholsäure, Arg, Lysophospatidylcholinacyl C16:0, Phospatidylcholinacylalkyl C36:3 |
| 21 | Octadecanoylcarnitin, Lanosterol, Desmosterol, Asp, Arachidonsäure, Lysophospatidylcholinacyl C17:0, Phospatidylcholinacylalkyl C36:4, Phospatidylcholinacylalkyl C36:5, Taurodeoxycholsäure, Taurin-konjugierte Taurochenodeoxycholsäure |
| 22 | Octadecanoylcarnitin, Lanosterol, Desmosterol, Asp, Arachidonsäure |
| 23 | Octadecanoylcarnitin, Lanosterol, Desmosterol, Asp |
| 24 | Octadecanoylcarnitin, Lanosterol, Desmosterol |
| 25 | Octadecanoylcarnitin, Lanosterol |
| 26 | Lanosterol, Desmosterol, Asp |
| 27 | Desmosterol, Asp, Arachidonsäure |
wobei bei Acylcarnitinen, Phosphatidylcholinen, und Lysophosphatidylcholinen, die Zahl, welche dem "C" folgt, die Anzahl der gesamten Kohlenstoffatome in der Fettsäure und/oder Alkylrest(e) repräsentiert, und die Zahl nach dem Doppelpunkt die Anzahl sämtlicher Doppelbindungen in dem Fettsäurerest und/oder Alkylrest(en) repräsentiert;
b) Messen wenigstens eines Parameters, welcher ausgewählt wird aus der Gruppe, bestehend aus: Vorliegen, Konzentration, Niveau oder Menge jeder einzelnen spezifischen Verbindung der Mehrzahl von Verbindungen in der Probe; Erhalten eines qualitativen und/oder quantitativen molekularen Musters aus der Gesamtheit der aus den Parametern erhaltenen Werte; und Speichern des erhaltenen Wertesatzes in einer Datenbank;
c) Kalibrieren der Werte durch Vergleichen mit klinisch bestätigten Schlaganfallpositiven und/oder klinisch bestätigten Schlaganfall-negativen Referenzparametern; und
d) Vergleichen der gemessenen Werte in der Probe mit den kalibrierten Werten, um abzuschätzen, ob der Patient Schlaganfall-positiv oder Schlaganfall-negativ ist.

2. Verfahren nach Anspruch 1, wobei der Schlaganfall ischämischen Schlaganfall und hämorrhagischen Schlaganfall umfasst.

3. Verfahren nach Anspruch 1 oder 2, wobei die quantitative Erfassung ein Erstellen eines Metabolomics-Profils umfasst, welches erhalten wird durch ein quantitatives Metabolomics-Profil-Analyseverfahren, welches umfasst: die Erzeugung von Intensitätsdaten zur Quantifizierung der Verbindungen mittels Massenspektrometrie (MS), insbesondere mittels Hochdurchsatzmassenspektrometrie, vorzugsweise mittels MS-Technologien wie Matrix-assistierter Laser Desorption/Ionisation (MALDI), Elektrosprayionisation (ESI), chemische Ionisation unter Atmosphärendruck (APCI), ¹H-, ¹³C- und/oder ³¹P- magnetische Kernresonanzspektroskopie (NMR), gegebenenfalls gekoppelt mit MS, Bestimmung der Metabolitenkonzentrationen unter Verwendung von MS-Technologien und/oder Verfahren gekoppelt mit Trennung, insbesondere Flüssigchromatographie (LC-MS), Gaschromatographie (GC-MS), oder Kapillarelektrophorese (CE-MS).

4. Verfahren nach irgendeinem der Ansprüche 1 to 3, wobei die Intensitätsdaten des Metabolomics-Profils mit einem Satz endogener Housekeeper-Metaboliten normalisiert werden durch Beziehen der erfassten Intensitäten der ausgewählten Schlaganfallspezifischen Zielmetaboliten auf die erfassten Intensitäten der Housekeeper-Metaboliten.

5. Verfahren nach Anspruch 4, wobei die Housekeeper Metaboliten ausgewählt werden aus der Gruppe, bestehend aus solchen Metaboliten, welche in Übereinstimmung mit statistischen Stabilitätsmaßen Stabilität zeigen, ausgewählt aus der Gruppe, bestehend aus: Variationskoeffizienten (CV) der Intensitätsrohdaten, Standardabweichung (SD) der logarithmischen Intensitätsdaten, Stabilitätsmaß (M) des geNorm - Algorithmus oder Stabilitätsmaßwert (rho) des NormFinder-Algorithmus.

6. Verfahren nach irgendeinem der Ansprüche 1 bis 5, wobei ein Panel von Referenzzielverbindungen oder Derivaten davon erstellt wird mittels:
a) mathematischem Vorverarbeiten von Intensitätswerten, welche zum Erzeugen der Metabolomics-Profile erhalten wurden, um technische Fehler zu verringern, welche den Messverfahren inhärent sind, welche verwendet wurden, die Metabolomics-Profile zu erzeugen;
b) Auswählen wenigstens eines geeigneten Klassifikationsalgorithmus aus der Gruppe, bestehend aus: logistischer Regression, (diagonal) linearer oder quadratischer Diskriminantenanalyse (LDA, QDA, DLDA, DQDA), Perceptron, geschrumpften Centroiden, regularisierter Diskriminantenanalyse (RDA), zufällige Wälder [random forests] (RF), neuronale Netzwerke (NN), Bayesische Netzwerke, versteckte Markov- Modelle, Support-Vektor-Maschinen (SVM), verallgemeinerte partielle kleinste Quadrate (GPLS), Verteilung um Medoide (PAM), inductive logische Programmierung (ILP), verallgemeinerte additive Modelle, Gauss'sche Prozesse, Regression mit regularisierten kleinsten Quadraten, selbstorganisierende Karten (SOM), recursive Verteilung und Regressionsbäume, K-nächste Nachbarn Klassifikatoren (K-NN), verschwommene [fuzzy] Klassifikatoren, Bagging, Boosting und naive Bayes; und Anwenden der ausgewählten Klassifikationsalgorithmen auf die vorverarbeiteten Daten von Schritt a);
c) wobei die Klassifikationsalgorithmen von Schritt b) mittels wenigstens eines Trainingsdatensatzes trainiert werden, welche vorverarbeitete Daten aus Subjekten enthalten, die in Klassen, entsprechend ihrer Wahrscheinlichkeit, eingeteilt sind, um eine entzündungsbedingte Hirnschädigung zu entwickeln, um eine Klassifikationsfunktion auszuwählen, welche die vorverarbeiteten Daten auf der Wahrscheinlichkeit abbildet;
d) Anwenden der trainierten Klassifikatoralgorithmen von Schritt c) auf einen vorverarbeiteten Datensatz eines Subjektes mit unbekannter Wahrscheinlichkeit für einen Schlaganfall, und Verwenden der trainierten Klassifikatoralgorithmen, um das Klassenetikett des Datensatzes vorherzusagen, um die Wahrscheinlichkeit eines Subjektes einen Schlaganfall zu erleiden, vorherzusagen.

7. Verfahren nach irgendeinem der Ansprüche 1 bis 6, wobei die prädiktiven Zielmetaboliten zur erleichterten und/oder empfindlicheren Erfassung mittels chemisch modifizierten Derivaten davon erfasst werden, wie beispielsweise Phenyisothiocyanaten für Aminosäuren.

8. Verwendung einer Mehrzahl von natürlich vorkommenden Metabolitenverbindungen zum Diagnostizieren eines Schlaganfalls aus einer Blutprobe eines Patienten in vitro innerhalb von 96 h nach dem Auftreten klinischer Symptome, wobei die Verbindungen ein Molekulargewicht von unter 1500 Dalton aufweisen und wobei die Mehrzahl der Verbindungen wenigstens einen Metabolitenset umfasst, der ausgewählt ist aus den Metabolitensets, welche ausgewählt werden aus der Gruppe, bestehend aus:
| Set Nr | Metabolitenverbindungen |
|---|---|
| 18 | Octadecanoylcarnitin, Lanosterol, Desmosterol, Asp, Arachidonsäure, Lysophospatidylcholinacyl C17:0, Phospatidylcholinacylalkyl C36:4, Phospatidylcholinacylalkyl C36:5, Taurodeoxycholsäure, Taurin-konjugierte Taurochenodeoxycholsäure, Taurolithocholsäuresulfat, Docosahexaensäure, Glycolithocholsäuresulfat, Thr, Glu, Taurocholsäure, Arg, Lysophospatidylcholinacyl C16:0, Phospatidylcholinacylalkyl C38:4, Phospatidylcholinacylalkyl C36:3, Lysophospatidylcholinacyl C18:0, Phospatidylcholinacylalkyl C38:5, Tetradecanoylcarnitin, Phospatidylcholinacylalkyl C40:1, Lysophospatidylcholinacyl C16:1 |
| 19 | Octadecanoylcarnitin, Lanosterol, Desmosterol, Asp, Arachidonsäure, Lysophospatidylcholinacyl C17:0, Phospatidylcholinacylalkyl C36:4, Phospatidylcholinacylalkyl C36:5, Taurodeoxycholsäure, Taurin-konjugierte Taurochenodeoxycholsäure, Taurolithocholsäuresulfat, Docosahexaensäure, Glycolithocholsäuresulfat, Thr, Glu, Taurocholsäure, Arg, Lysophospatidylcholinacyl C16:0, Phospatidylcholinacylalkyl C38:4, Phospatidylcholinacylalkyl C36:3 |
| 20 | Octadecanoylcarnitin, Lanosterol, Desmosterol, Asp, Arachidonsäure, Phospatidylcholinacylalkyl C36:4, Taurodeoxycholsäure, Taurin-konjugierte Taurochenodeoxycholsäure, Taurolithocholsäuresulfat, Docosahexaensäure, Glycolithocholsäuresulfat, Thr, Glu, Taurocholsäure, Arg, Lysophospatidylcholinacyl C16:0, Phospatidylcholinacylalkyl C36:3 |
| 21 | Octadecanoylcarnitin, Lanosterol, Desmosterol, Asp, Arachidonsäure, Lysophospatidylcholinacyl C17:0, Phospatidylcholinacylalkyl C36:4, Phospatidylcholinacylalkyl C36:5, Taurodeoxycholsäure, Taurin-konjugierte Tau rochenodeoxycholsäure |
| 22 | Octadecanoylcarnitin, Lanosterol, Desmosterol, Asp, Arachidonsäure |
| 23 | Octadecanoylcarnitin, Lanosterol, Desmosterol, Asp |
| 24 | Octadecanoylcarnitin, Lanosterol, Desmosterol |
| 25 | Octadecanoylcarnitin, Lanosterol |
| 26 | Lanosterol, Desmosterol, Asp |
| 27 | Desmosterol, Asp, Arachidonsäure |
wobei bei Acylcarnitinen, Phosphatidylcholinen und Lysophosphatidylcholinen die Zahl, die auf "C" folgt, die Anzahl sämtlicher Kohlenstoffatome in der Fettsäure und/oder Alkylrest repräsentiert, und die Zahl nach dem Doppelpunkt die Anzahl sämtlicher Doppelbindungen in dem Fettsäurerest und/oder Alkylrest repräsentiert.

## Revendications

1. Procédé de diagnostic d'attaque (accident vasculaire cérébral) chez des patients dans les 96 h après la survenue de symptômes cliniques,
**caractérisé par**
la détection quantitative *in vitro* dans au moins un échantillon de sang d'un patient d'une pluralité de composés métaboliques qui sont spécifiques d'une attaque, et ayant un poids moléculaire inférieur à 1 500 Daltons comprenant les étapes de :
a) sélection de ladite pluralité de composés métaboliques comprenant au moins l'un des ensembles de métabolites choisis dans le groupe consistant en :
| Ensemble N° | Composés métaboliques |
|---|---|
| 18 | Octadécanoylcarnitine, Lanostérol, Desmostérol, Asp, Acide arachidonique, Lysophosphatidylcholine acyle C17:0, Phosphatidylcholine acyl-alkyle C36:4, Phosphatidylcholine acyl-alkyle C36:5, Acide taurodésoxycholique, Acide taurochénodésoxycholique conjugué de taurine, Sulfate d'acide taurolithocholique, Acide docosahexaénoïque, Sulfate d'acide glycolithocholique, Thr, Glu, Acide taurocholique, Arg, Lysophosphatidylcholine acyle C16:0, Phosphatidylcholine acyl-alkyle C38:4, Phosphatidylcholine acyl-alkyle C36:3, Lysophosphatidylcholine acyle C18:0, Phosphatidylcholine acyl-alkyle C38:5, Tétradécanoylcarnitine, Phosphatidylcholine acyl-alkyle C40:1, Lysophosphatidylcholine acyle C16:1 |
| 19 | Octadécanoylcarnitine, Lanostérol, Desmostérol, Asp, Acide arachidonique, Lysophosphatidylcholine acyle C17:0, Phosphatidylcholine acyl-alkyle C36:4, Phosphatidylcholine acyl-alkyle C36:5, Acide taurodésoxycholique, Acide taurochénodésoxycholique conjugué de taurine, Sulfate d'acide taurolithocholique, Acide docosahexaénoïque, Sulfate d'acide glycolithocholique, Thr, Glu, Acide taurocholique, Arg, Lysophosphatidylcholine acyle C16:0, Phosphatidylcholine acyl-alkyle C38:4, Phosphatidylcholine acyl-alkyle C36:3 |
| 20 | Octadécanoylcarnitine, Lanostérol, Desmostérol, Asp, Acide arachidonique, Phosphatidylcholine acyl-alkyle C36:4, Acide taurodésoxycholique, Acide taurochénodésoxycholique conjugué de taurine, Sulfate d'acide taurolithocholique, Acide docosahexaénoïque, Sulfate d'acide glycolithocholique, Thr, Glu, Acide taurocholique, Arg, Lysophosphatidylcholine acyle C16:0, Phosphatidylcholine acyl-alkyle C36:3 |
| 21 | Octadécanoylcarnitine, Lanostérol, Desmostérol, Asp, Acide arachidonique, Lysophosphatidylcholine acyle C17:0, Phosphatidylcholine acyl-alkyle C36:4, Phosphatidylcholine acyl-alkyle C36:5, Acide taurodésoxycholique, Acide taurochénodésoxycholique conjugué de taurine |
| 22 | Octadécanoylcarnitine, Lanostérol, Desmostérol, Asp, Acide arachidonique |
| 23 | Octadécanoylcarnitine, Lanostérol, Desmostérol, Asp |
| 24 | Octadécanoylcarnitine, Lanostérol, Desmostérol |
| 25 | Octadécanoylcarnitine, Lanostérol |
| 26 | Lanostérol, Desmostérol, Asp |
| 27 | Desmostérol, Asp, Acide arachidonique |
dans lequel dans les acylcarnitines, les phosphatidylcholines, et les lysophosphatidylcholines, le nombre suivant « C » représente le nombre d'atomes de carbone total dans le ou les résidus d'acide gras et/ou d'alkyle, et le nombre après les deux points représente le nombre de doubles liaisons total dans le résidu d'acide gras et/ou le(s) résidu(s) d'alkyle ;
b) mesure d'au moins l'un des paramètres choisis dans le groupe consistant en : présence, concentration, niveau ou quantité de chaque composé spécifique de ladite pluralité de composés dans ledit échantillon ; obtention d'un motif moléculaire qualitatif et/ou quantitatif à partir de la totalité des valeurs obtenues à partir desdites mesures de paramètre ; et stockage dudit ensemble de valeurs obtenu dans une base de données ;
c) étalonnage desdites valeurs en comparant des paramètres de référence confirmant l'attaque confirmé cliniquement et/ou infirmant l'attaque confirmé cliniquement ; et
d) comparaison desdites valeurs mesurées dans l'échantillon avec les valeurs étalonnées, afin d'évaluer si le patient a subi une attaque ou n'a pas subi d'attaque.

2. Procédé selon la revendication 1, dans lequel l'attaque comprend un accident ischémique cérébral et une attaque d'apoplexie hémorragique.

3. Procédé selon la revendication 1 ou 2, dans lequel ladite détection quantitative comprend l'établissement d'un profil métabolomique qui est obtenu par un procédé d'analyse quantitative de profils métabolomiques comprenant la génération de données d'intensité pour la quantification desdits composés par spectrométrie de masse (MS), en particulier par spectrométrie de masse à haut débit, de préférence par des technologies MS telles que la désorption/ionisation laser assistée par matrice (MALDI), la ionisation par électronébulisation (ESI), ionisation chimique à pression atmosphérique (APCI), spectrométrie de résonance magnétique nucléaire (RMN) ¹H-, ¹³C- et/ou ³¹P-, facultativement couplé à une MS, détermination de concentrations de métabolites grâce à l'utilisation de technologies de MS et/ou de procédés couplés à une séparation, en particulier la chromatographie liquide (LC-MS), la chromatographie gazeuse (GC-MS), ou l'électrophorèse capillaire (CE-MS).

4. Procédé selon l'une quelconque des revendications 1 à 3, dans lequel des données d'intensité dudit profil métabolomique sont normalisées avec un ensemble de métabolites domestiques endogènes en reliant des intensités détectées des métabolites cibles sélectionnés sont spécifiques d'une attaque à des intensités détectées desdits métabolites de domestiques.

5. Procédé selon la revendication 4, dans lequel lesdits métabolites domestiques sont choisis dans le groupe consistant en ces métabolites qui présentent une stabilité selon des mesures de stabilité statistique qui sont choisies dans le groupe consistant en un coefficient de variation (CV) de données d'intensité brutes, un écart-type (SD) de données d'intensité logarithmiques, une mesure de stabilité (M) d'algorithme geNorm ou une valeur de mesure de stabilité (rho) d'algorithme NormFinder.

6. Procédé selon l'une quelconque des revendications 1 à 5, dans lequel un panel de composés cibles de référence ou de dérivés de ceux-ci est établi par :
a) le prétraitement mathématique de valeurs d'intensité obtenues pour générer les profils métabolomiques afin de réduire des erreurs techniques qui sont inhérentes aux procédures de mesure utilisées pour générer les profils métabolomiques ;
b) la sélection d'au moins un algorithme de classification approprié dans le groupe consistant en la régression logistique, l'analyse discriminante linéaire ou quadratique (diagonale) (LDA, QDA, DLDA, DQDA), le perceptron, une analyse discriminante régularisée par centroïdes rétrécis (RDA), les forêts aléatoires (RF), les réseaux neuronaux (NN), les réseaux bayésiens, les modèles de Markov cachés, les machines à vecteur de support (SVM), les moindres carrés partiels généralisés (GPLS), le partitionnement autour de médoïdes (PAM), la programmation par logique inductive (ILP), les modèles additifs généralisés, les processus gaussiens, la régression des moindres carrés régularisés, des cartes d'auto-organisation (SOM), les arbres de partitionnement récursif et de régression, des classificateurs des K-plus proches voisins (K-NN), les classificateurs flous, l'ensachage, l'amplification, et la classification bayésienne élémentaire ; et l'application dudit algorithme classificateur sélectionné auxdites données prétraitées de l'étape a) ;
c) lesdits algorithmes classificateurs de l'étape b) étant entraînés sur au moins un ensemble de données d'apprentissage contenant des données prétraitées provenant de sujets qui sont divisés en classes selon leur probabilité de développer une lésion cérébrale liée à une inflammation, afin de sélectionner une fonction de classificateur pour mapper lesdites données prétraitées par rapport à ladite probabilité ;
d) l'application desdits algorithmes classificateurs entraînés de l'étape c) à un ensemble de données prétraitées d'un sujet avec une probabilité inconnue d'attaque, et l'utilisation des algorithmes classificateurs entraînés pour prédire l'étiquette de classe dudit ensemble de données afin de prédire la probabilité qu'a un sujet de subir une attaque.

7. Procédé selon l'une quelconque des revendications 1 à 6, dans lequel lesdits métabolites cibles prédictifs pour une détection plus aisée et/ou plus sensible sont détectés au moyen de dérivés modifiés chimiquement de ceux-ci, tels que des phénylisothiocyanates pour des acides aminés.

8. Utilisation d'une pluralité de composés métaboliques d'origine naturelle pour diagnostiquer une attaque à partir d'un échantillon de sang de patient *in vitro* dans les 96 h après la survenue de symptômes cliniques, dans laquelle lesdits composés ont un poids moléculaire inférieur à 1 500 Daltons, et dans laquelle ladite pluralité de composés comprend au moins l'un des ensembles de métabolites choisis dans le groupe consistant en :
| Ensemble N° | Composés métaboliques |
|---|---|
| 18 | Octadécanoylcarnitine, Lanostérol, Desmostérol, Asp, Acide arachidonique, Lysophosphatidylcholine acyle C17:0, Phosphatidylcholine acyl-alkyle C36:4, Phosphatidylcholine acyl-alkyle C36:5, Acide taurodésoxycholique, Acide taurochénodésoxycholique conjugué de taurine, Sulfate d'acide taurolithocholique, Acide docosahexaénoïque, Sulfate d'acide glycolithocholique, Thr, Glu, Acide taurocholique, Arg, Lysophosphatidylcholine acyle C16:0, Phosphatidylcholine acyl-alkyle C38:4, Phosphatidylcholine acyl-alkyle C36:3, Lysophosphatidylcholine acyle C18:0, Phosphatidylcholine acyl-alkyle C38:5, Tétradécanoylcarnitine, Phosphatidylcholine acyl-alkyle C40:1, Lysophosphatidylcholine acyle C16:1 |
| 19 | Octadécanoylcarnitine, Lanostérol, Desmostérol, Asp, Acide arachidonique, Lysophosphatidylcholine acyle C17:0, Phosphatidylcholine acyl-alkyle C36:4, Phosphatidylcholine acyl-alkyle C36:5, Acide taurodésoxycholique, Acide taurochénodésoxycholique conjugué de taurine, Sulfate d'acide taurolithocholique, Acide docosahexaénoïque, Sulfate d'acide glycolithocholique, Thr, Glu, Acide taurocholique, Arg, Lysophosphatidylcholine acyle C16:0, Phosphatidylcholine acyl-alkyle C38:4, Phosphatidylcholine acyl-alkyle C36:3 |
| 20 | Octadécanoylcarnitine, Lanostérol, Desmostérol, Asp, Acide arachidonique, Phosphatidylcholine acyl-alkyle C36:4, Acide taurodésoxycholique, Acide taurochénodésoxycholique conjugué de taurine, Sulfate d'acide taurolithocholique, Acide docosahexaénoïque, Sulfate d'acide glycolithocholique, Thr, Glu, Acide taurocholique, Arg, Lysophosphatidylcholine acyle C16:0, Phosphatidylcholine acyl-alkyle C36:3 |
| 21 | Octadécanoylcarnitine, Lanostérol, Desmostérol, Asp, Acide arachidonique, Lysophosphatidylcholine acyle C17:0, Phosphatidylcholine acyl-alkyle C36:4, Phosphatidylcholine acyl-alkyle C36:5, Acide taurodésoxycholique, Acide taurochénodésoxycholique conjugué de taurine |
| 22 | Octadécanoylcarnitine, Lanostérol, Desmostérol, Asp, Acide arachidonique |
| 23 | Octadécanoylcarnitine, Lanostérol, Desmostérol, Asp |
| 24 | Octadécanoylcarnitine, Lanostérol, Desmostérol |
| 25 | Octadécanoylcarnitine, Lanostérol |
| 26 | Lanostérol, Desmostérol, Asp |
| 27 | Desmostérol, Asp, Acide arachidonique |
dans laquelle dans les acylcarnitines, les phosphatidylcholines, et les lysophosphatidylcholines, le nombre suivant « C » représente le nombre d'atomes de carbone total dans le résidu d'acide gras et/ou d'alkyle, et le nombre après les deux points représente le nombre de doubles liaisons total dans le résidu d'acide gras et/ou le résidu d'alkyle.
